# EUROPÄISCHE PATENTSCHRIFT

(11) **EP 0 332 581 B1**
(45) Veröffentlichungstag und Bekanntmachung des Hinweises auf die Patenterteilung: **11.12.1996**
(21) Anmeldenummer: 89810162.1
(22) Anmeldetag: 02.03.1989
(51) Int. Cl.: A01G 7/00, C12N 5/00, A01H 1/00, C12N 15/81

(54) **Regeneration von fertilen Gramineen-Pflanzen aus der Unterfamilie Pooideae ausgehend von Protoplasten**
Regeneration of fertile graminaceous plants of the subfamily pooideae from protoplasts
Régénération de plantes graminées fertiles de la sous-famille des pooideae à partir de protoplastes

(30) Priorität: 08.03.1988 US 165665
(43) Veröffentlichungstag der Anmeldung: 13.09.1989
(73) Patentinhaber: CIBA-GEIGY AG, 4002 Basel (CH)
(72) Erfinder: Horn, Michael E., WoodlandCalifornia 95695 (US); Harms, Christian T., Chapel HillNorth Carolina 27514 (US); Shillito, Raymond D., Dr., Chapel HillNorth Carolina 27514 (US)

(56) Entgegenhaltungen:
- EP-A- 0 176 162
- EP-A- 0 257 472
- EP-A- 0 331 083
- BIOLOGICAL ABSTRACTS, Zusammenfassung Nr. 35:113831, accession Nr. biosis 88:494996; S.J. DALTON 1988, 137-140
- BIOLOGICAL ABSTRACTS, Band 79, 1985, Zusammenfassung Nr. 18364; D.J. GRAY et al.: "Somatic embryogenesis is suspension and suspension-derived callus cultures of Dactylis glomerata", & PROTOPLASMA 122(3): 196- 202, 1984
- MOL. GEN. GENET., Band 199, 1985, Seiten 183-188, Springer-Verlag; I. POTRYKUS et al.: "Direct gene transfer to cells of a graminaceous monocot"
- BIOLOGICAL ABSTRACTS, Band 83, 1987, Zusammenfassung Nr.122226, Philadelphia, US; S.E. MADDOCK: "Suspension and protoplast culture of hexaploid wheat (Triticum aestivum L.)", & PLANT CELL. REP 6(1): 23- 26, 1987
- BIOLOGICAL ABSTRACTS, Band 86, 1988, Zusammenfassung Nr. 33950; S.J. DALTON: 170-175, 1988
- BIOLOGICAL ABSTRACTS, Band 87, 1989, Zusammenfassung Nr. 43934; M.E. HORN et al.: "Plant regeneration from protoplasts of embryogenic suspension cultures of orchardgrass (Dactylis glomerata L.)", & PLANT CELL REP., 7(6): 371-274, 1988
- CHEMICAL ABSTRACTS, Band 110, 1989, Seite 208, Zusammenfassung Nr. 89869a, Columbus, Ohio, US; M.E. HORN et al.: "Transgenic plants of orchard grass (Dactylis glomerata L.) from protoplasts", & PLANT CELL REP. 1988, 7(7), 469-72
- BIOLOGICAL ABSTRACTS, Band 30, Zusammenfassung Nr. 4959; P.J. DALE et al.: "The development of protoplast systems for genetic exchange in forage grasses",& ILLUS. 0(0), 1985, 164-165
- Cocking, E.C. and Davey, M.R.,1987, Science vol. 236, pp.1259-1262

## Beschreibung

Die vorliegende Erfindung betrifft Gräser aus der Unterfamilie (Subfamilia) Pooideae, die aus Protoplasten, aus Protoplasten mit regenerierten Zellwänden (pflanzliche Zellen) oder aus Kalli, die von Protoplasten abstammen, regeneriert werden sowie allgemein anwendbare Verfahren zur Regeneration dieser Pflanzen. Ein weiterer Gegenstand dieser Erfindung betrifft embryogene Zellkulturen (Suspensionskulturen oder Kalluskulturen) sowie Kalli, die als Ausgangsmaterial für Protoplasten dienen, die dann ihrerseits zu ganzen Pflanzen regeneriert werden können. Ebenso umfasst von dieser Erfindung sind Verfahren für die Herstellung der oben erwähnten embryogenen Zellkulturen, die Kältekonservierung (Kryopräservation) besagter embryogener Zellkulturen und der embryogenen Kalli sowie transgene Pflanzen aus der Unterfamilie Pooideae, die aus genetisch modifizierten Protoplasten regeneriert werden.

Die Mehrzahl der Pflanzen, von denen die menschliche Ernährung in erster Linie abhängig ist, gehört zu einer Gruppe von Pflanzen, die unter dem Sammelbegriff Gramineen bekannt sind. Die Gramineae (Poaceae) sind aus kommerzieller Sicht heraus betrachtet die bedeutendste Familie innerhalb der Klasse der monokotylen Pflanzen. Zu den Gramineen gehören beispielsweise die folgenden Unterfamilien und Gattungen:

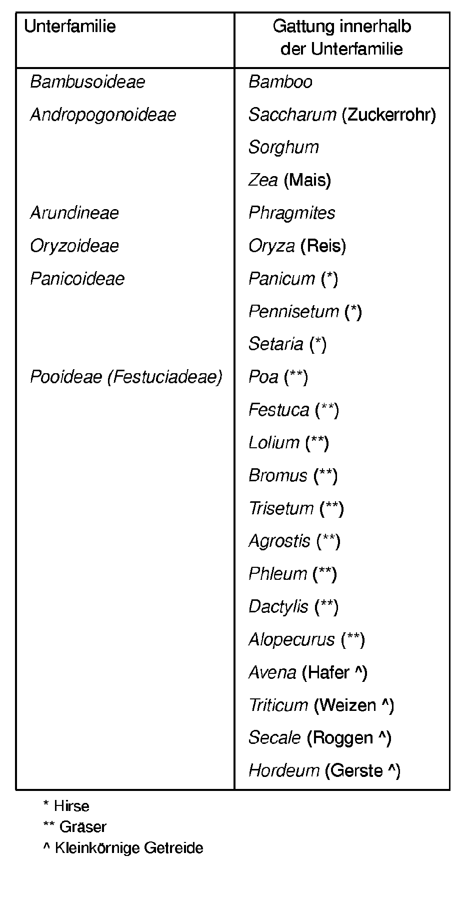

Innerhalb der Unterfamilien der Gramineen bilden die Pooideae eine unter ökonomischen Gesichtspunkten sehr bedeutende Pflanzengruppe. Dazu gehören z.B. auch die beiden nahe verwandten Untergruppen der Gräser und der kleinkörnigen Getreide.

Interessanterweise sind es gerade die Pflanzen aus dieser Unterfamilie der Pooideae, die sich am schwierigsten mit Hilfe wissenschaftlicher Methoden manipulieren lassen. Entsprechend ist bis heute kein generelles Verfahren bekannt, das die Regeneration von Pflanzen, von fertilen Pflanzen oder von transgenen Pflanzen mit stabil eingebauter exogener DNA aus der Unterfamilie der Pooideae ausgehend von Protoplasten erlaubt, obwohl eine solche Regeneration aus kultivierten Protoplasten z.B. für die Anwendung der somatischen Hybridisierung und für die Herstellung transgener Pflanzen mit Hilfe des direkten Gentransfers Voraussetung ist. Der gegenwärtige Kenntnisstand auf dem Gebiet der Getreidetransformation wurde kürzlich von Cocking und Davey, 1987 in einem Übersichtsartikel zusammenfassend dargestellt.

Eine Beschreibung von Ausgangsmaterial für Getreidekulturen, von Protoplasten sowie von Verfahren zur Isolierung von Getreideprotoplasten und deren Eigenschaften findet sich beispielsweise in folgender Publikation: "Cereal Tissue and Cell Culture"; Bright SWJ and Jones MGK, (eds) (1985) Nijhoff M; Junk W , Dordrecht.

Eine stabile Transformation von Gramineen konnte bereits durch eine chemisch und elektrisch stimulierte Aufnahme von DNA in Protoplasten erreicht werden ("Direkter Gentransfer";Potrykus et al, 1985; Lörz et al, 1985; Fromm et al, 1986), wohingengen eine Regeneration von ganzen Pflanzen, ausgehend von den in diesen Studien verwendeten Zellinien, nicht möglich war.

Bisher konnten nur solche Pflanzen aus der Gruppe der Gramineen erfolgreich regeneriert werden, die nicht der Unterfamilie der Pooideae angehören. Abdullah et al,(1986) berichten beispielsweise über eine effiziente Pflanzenregeneration durch somatische Embryogenese ausgehend von Reisprotoplasten (Unterfamilie Oryzoideae). Auch Yamada et al, (1986) beschreiben Pflanzenregeneration beim Reis ausgehend von Kalli, die sich von Protoplasten ableiten. Rhodes et al, (1988) beschreiben die Regeneration von nicht-fertilen Maispflanzen. Cocking und Davey, (1987) diskutieren den gegenwärtigen Kenntnisstand auf dem Gebiet des Gentransfers in Getreidepflanzen.

Die Regeneration von Gramineen-Pflanzen aus der Unterfamilie Pooideae ausgehend von Gewebekulturen ist bekannt. Hanning et al, (1982) beschreiben die Embryo- und Keimlingsentwicklung bei Dactylis glomerata L., ausgehend von Kallusgewebe, das sich aus Blattsegmenten entwickelt.

Im folgenden sind beispielhaft weitere Pflanzen aus der Unterfamilie Pooideae aufgeführt, deren Regeneration aus kultivierten Zellen in den folgenden Artikeln beschrieben ist:
Lolium rigidum (Skene et al, 1983); Lolium perenne, Lolium multiflorum (Ahloowalia, 1975); Lolium multiflorum, Festuca arundinacea (Kasperbauer et al, 1979); Alopecurus arundinaceus, Agropyron crystatum, Stipa viridula, Bromus inermis, Agropyron smithii (Lo et al, 1980) und Agrostis palustris (Krans et al, 1982).

Eine weitere Übersicht über Gewebekulturen bei Futtergräsern findet sich bei Ahloowalia, (1984).

In den oben angegebenen Fällen geht die Regeneration der Pooideae jedoch nicht von dem gleichen Ausgangsmaterial aus, das in der vorliegenden Erfindung offenbart ist, sondern von anderen Zellkulturtypen. In keinem der oben angegebenen Beispiele konnte nachgewiesen werden, dass die Regeneration de-novo über eine somatische Embryogenese erfolgt. Die oben zitierten Referenzen beinhalten darüberhinaus keine Angaben über die Isolierung und Kultivierung von Protoplasten oder über die Regeneration von Pflanzen aus Protoplasten.

Somit existiert trotz des grossen Interesses an einer genetischen Transformation und Regeneration von Gramineen aus der Unterfamilie Pooideae bis zum jetzigen Zeitpunkt kein in-vitro Verfahren, das eine erfolgreiche Regenertaion von Pflanzen oder fertilen Pflanzen aus Protoplasten, die gegebenenfalls transformiert sein können, erlaubt (Cocking and Davey, 1987).

Bisher sind alle Untersuchungen und Anstrengungen in dieser Richtung erfolglos geblieben, d.h. sie führten entweder nur zu Embryonen oder aber zu nicht lebensfähigen Pflänzchen, die bereits in einer frühen Entwicklungsphase abstarben und daher nicht erfolgreich in Erde überführt werden konnten (Ahloowalia, 1984)

Somit steht der Öffentlichkeit bis heute kein Verfahren zur Verfügung, das die Herstellung von Protoplasten aus der Unterfamilie Pooideae erlaubt, die zu einer Differenzierung zu ganzen Pflanzen und vorzugsweise zu ganzen fertilen Pflanzen in der Lage sind, geschweige denn ein Verfahren zur Regeneration von Pflanzen aus der Unterfamilie Pooideae ausgehend von Protoplasten oder von Kalli, die von Protoplasten abstammen.

Diese und andere Aufgaben konnten jetzt im Rahmen der vorliegenden Erfindung gelöst werden, die ein Verfahren zur Herstellung von Protoplasten offenbart, welche in der Lage sind Zell- und Kalluskolonien auszubilden. Die Protoplasten können, sofern dies gewünscht wird, transformiert werden, wobei die entstehenden Kalli in der Lage sind zu ganzen Pflanzen (Pooideae) zu regenerieren. Dieses Verfahren zur Herstellung von Protoplasten, die in der Lage sind sich zu teilen und Kallus zu entwickeln, der dann zu ganzen Pflanzen regeneriert werden kann, erfordern neuartige embryogene Zellkulturen (Suspensions- oder Kalluskulturen) oder Embryonen als Ausgangsmaterial. Diese embryogenen Zellkulturen und Embryonen sowie Verfahren zu deren Herstellung und Identifizierung werden in der vorliegenden Anmeldung beschrieben und sind ebenfalls ein Bestandteil dieser Erfindung.

Als Ausgangsmaterial für Protoplasten kann darüberhinaus embryogener Kallus verwendet werden, von dem sich die Suspensionen ableiten. Dieser Kallus sowie die Suspensionen, Embryonen und Verfahren zu deren Herstellung und Identifizierung werden in der vorliegenden Anmeldung beschrieben und sind ebenfalls ein Bestandteil dieser Erfindung.

Diese embryogenen Kulturen bilden die Quelle für Protoplasten, die mit exogener DNA transformiert werden können und die zur Zellteilung und zur Ausbildung von Kallus in der Lage sind. Dieser Kallus kann dann zu ganzen Pflanzen, einschliesslich ganzer fertiler Pflanzen, die in Erde wachstumsfähig sind, regeneriert werden.

Zu dem Zeitpunkt, als diese Erfindung gemacht wurde, liess sich aus dem Stand der Technik nicht ableiten, das es möglich ist fertile Gramineen, aus der Unterfamilie Pooideae ausgehend von Protoplasten oder aber von Zellen oder Kalli, die von Protoplasten abstammen, zu regenerieren. Noch weniger vorhersehbar war, dass Protoplasten von Pflanzen aus der Unterfamilie Pooideae, die exogene DNA stabil in ihr Genom eingebaut enthalten, ebenfalls zu fertilen transgenen Pflanzen, regeneriert werden können.

Diese Erfindung betrifft daher in erster Linie embryogene Zellkulturen (Suspensions- oder Kalluskulturen), die sich von Gramineen aus der Unterfamilie Pooideae ableiten, von denen Protoplasten isoliert werden können, wobei besagte Protoplasten zunächst Zellwände regenerieren, sich teilen und anschliessend einen Kallus ausbilden, der zu ganzen Pflanzen, einschliesslich ganzer fertiler Pflanzen, regeneriert werden kann, dadurch erhältlich, dass man
(a) Gewebe aus geeigneten Teilen von Pflanzen aus der Unterfamilie Pooideae isoliert;
(b) dieses Gewebe in einem Medium kultiviert, das in der Lage ist, die Ausbildung eines embryogenen Kallus oder von Embryonen zu induzieren;
(c) von besagtem Kallus und von besagten Embryonen periodisch Folgekulturen auf frischem Medium anlegt, indem man kleine Zellkluster mit cytoplasmareichen Zellen in frisches Medium überträgt, das in der Lage ist, eine kontinuierliche Proliferation in Gang zu halten; und
(d) nach 0 bis 500 Übertragungen (Transfers) embryogene Zellkluster mit einer Grösse von 150 /-Lm bis 2000 µm isoliert.

Darüberhinaus umfasst die vorliegende Erfindung regenerierte Gramineen- Pflanzen aus der Unterfamilie Pooideae sowie deren Vermehrungsgut, die sich von Protoplasten oder Pflanzenzellen ableiten, die exogene DNA in ihr Genom eingebaut enthalten die in Pflanzen exprimierbar ist. Unter Vermehrungsgut ist im Rahmen dieser Erfindung jegliches pflanzliche Material zu verstehen, das sich sexuell oder asexuell oder aber in vitro oder in vivo vermehren lässt, wobei Protoplasten, Zellen, Kalli, Gewebe, Organe, Zygoten, Embryonen, Pollen oder Samen, die von transgenen Pflanzen aus der Unterfamilie Pooideae erhalten werden können, bevorzugt sind. Einen weiteren Gegenstand dieser Erfindung bildet die Nachkommenschaft besagter Pflanzen aus der Unterfamilie Pooideae, sowie Mutanten und Varianten davon, einschliesslich derjenigen, die sich von Pflanzen ableiten, welche durch somatische Zellfusion, genetische Modifikation oder Mutantenselektion erhalten wurden.

Diese Erfindung betrifft darüberhinaus ein Verfahren zur Herstellung von Protoplasten und Pflanzenzellen aus Pflanzen der Unterfamilie Pooideae, die zu ganzen fertilen Pflanzen regeneriert werden können, weiterhin ein Verfahren zur Herstellung von Kalli, die sich von besagten Protoplasten oder Pflanzenzellen ableiten und die zur Regeneration ganzer fertiler Pflanzen, befähigt sind. Desweiteren betrifft die vorliegenden Erfindung ein Verfahren zur Regeneration von Pflanzen der Unterfamilie Pooideae ausgehend von besagten Kalli. Diese Verfahren. Sind dadurch gekennzeichnet das man von embryogenen Kallus oder Embryonen periodisch Folgekulturen auf frisches Medium anlegt, indem man kleine Zellkluster mit cytoplasmareichen Zellen in frisches Medium überträgt, das in der Lage ist, eine Kontinuierliche Proliferation in Gang zu halten. Außerdem werden nach 0-500 Übertragungen (Transfers) embryogene Zellkluster mit einer Grösse von 150-2000 µm isoliert.

Diese und weitere Gegenstände der vorliegenden Erfindung werden in der nachfolgenden detaillierten Beschreibung offenbart.

### Abbildungen

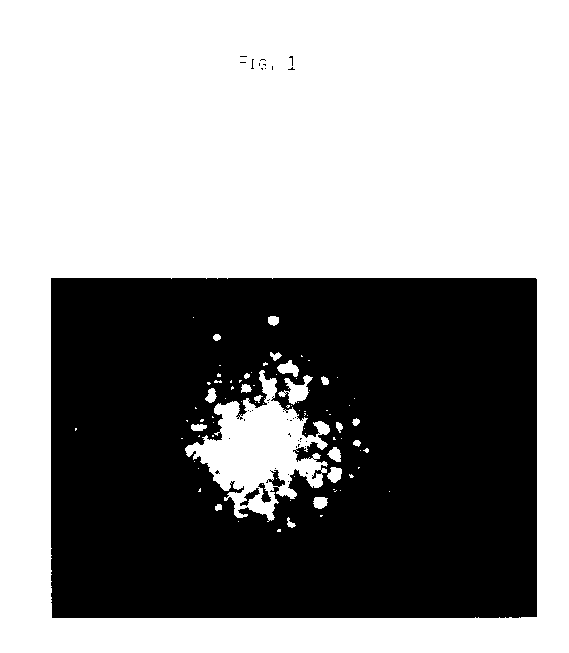

Figur 1: Die Abblidung zeigt Kolonien von Dactylis glomerata L., die von Protoplasten abstammen und die in einem Agaroseeinschluss wachsen, der in einem Flüssigmedium suspendiert ist.

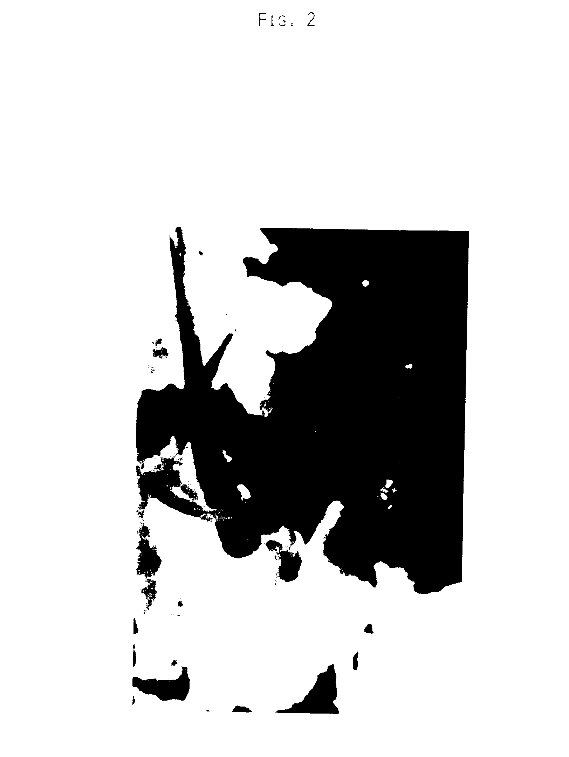

Figur 2: Die Abbildung zeigt eine Jungpflanze, die sich aus einem von Protoplasten abgeleiteten Dactylis glomerata L. Kallus entwickelt, der auf SH-0 Medium wächst.

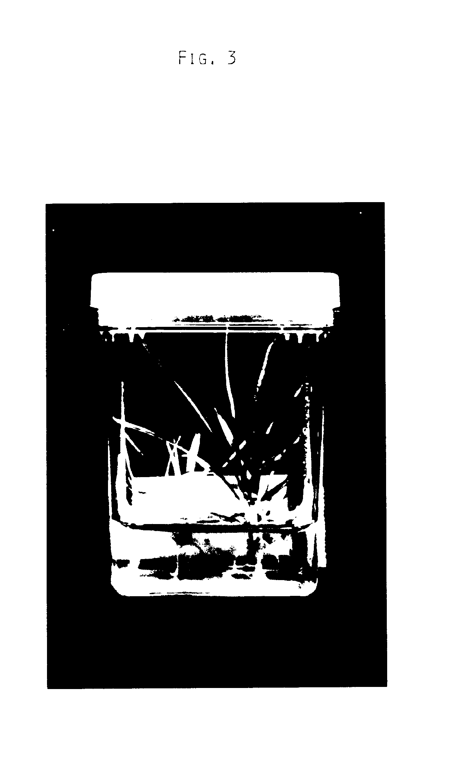

Figur 3: Die Abbildung zeigt einen Behälter mit einer bewurzelten Jungpflanze, die sich aus einem auf SH-0 Medium wachsenden Dactylis glomerata L. Kallus heraus entwickelt, der sich von Protoplasten ableitet.

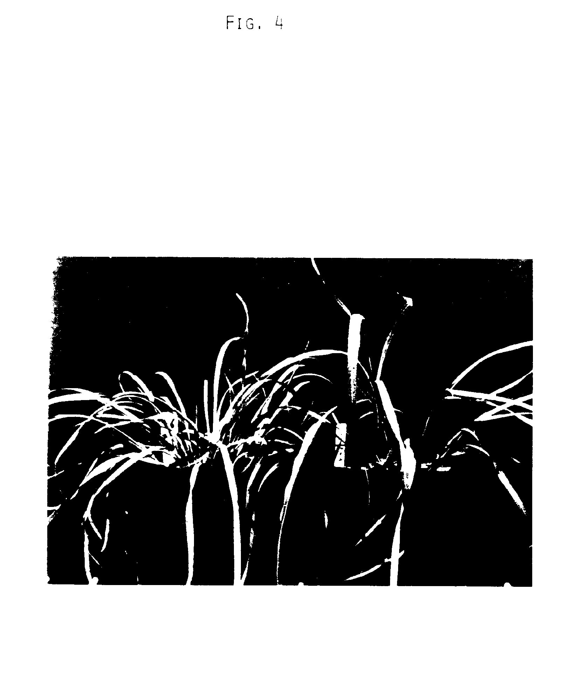

Figur 4: Die Abbildung zeigt eine Dactylis glomerata L. Pflanze, die aus Protoplasten regeneriert wurde (links) zusammen mit einer Wildtyp Dactylis glomerata L. Pflanze (rechts).

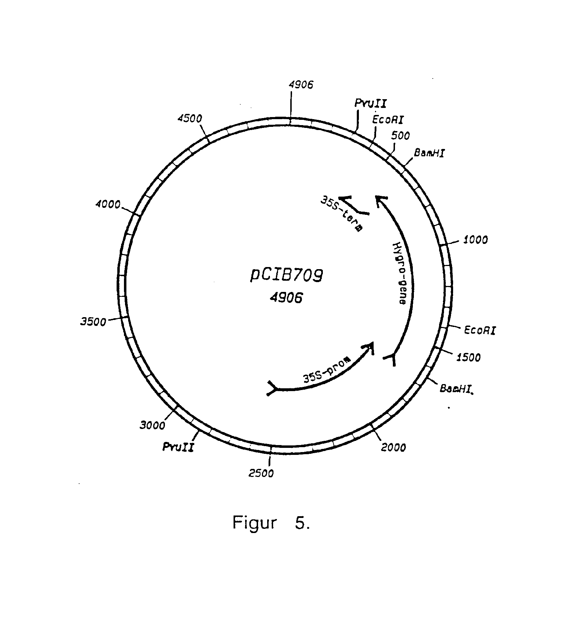

Figur 5: Die Abbildung zeigt das Plasmid pCIB709, das für die Transformation von Dactylis glomerata L. Protoplasten verwendet werden kann, um diesen eine Resistenz gegen Hygromycin zu verleihen. Das Plasmid pCIB709 wurde gemäss den Bestimmungen des Budapester Vertrages bei der 'American Type Culture Collection' in Rockville, Maryland, USA unter der Hinterlegungsnummer ATCC 40428 hinterlegt. Das Hinterlegungsdatum ist der 12 Februar 1988. 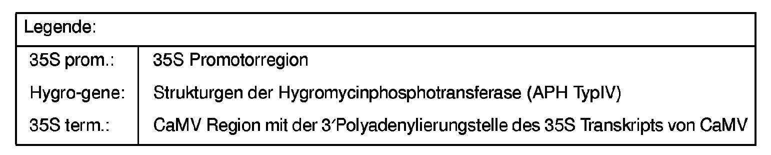

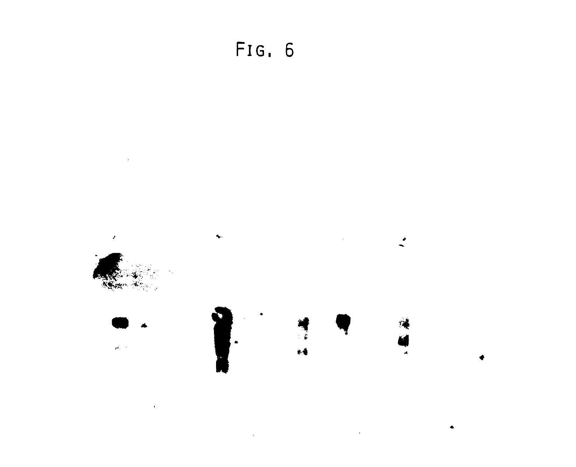

Figur 6: Die Abbildung zeigt die 'Southern blot' Analyse der DNA verschiedener Dactylis glomerata L. Kalli, die nach der Transformation von Protoplasten mit dem Plasmid pCIB709 aus diesen gewonnen wurden. Die Analyse wurde mit Hilfe des Xbal-Sstl Fragmentes, das als molekulare Sonde diente, durchgeführt.

Reihe 1,2: 10 ng und 2 ng pCIB709 geschnitten mit der Restriktionsendonuklease BamHl
Reihe 4-8: BamHI geschnittene DNA aus Dactylis glomerata Kallus-Kulturen, die sich von Protoplasten ableiten, welche zuvor mit pCIB709 transformiert wurden.
Reihe 9,17: BamHl geschnittene DNA aus nicht-transformiertem, von Protoplasten abgeleitetem Dactylis glomerata L. Kallus.
Reihe 10-13: BamHl geschnittene DNA aus Dactylis glomerata L. Kallus-Kulturen, die sich von Protoplasten ableiten, die zuvor mit pCIB709 transformiert wurden.
Reihe 14: BamHl geschnittene DNA aus Dactylis glomerata L. Kallus-Kulturen, die sich von Protoplasten ableiten, die zuvor mit pCIB709 transformiert wurden.
Reihe 15,16: BamHl geschnittene DNA aus Dactylis glomerata L. Kallus-Kulturen, die sich von Protoplasten ableiten, die zuvor mit pCIB709 transformiert wurden.
Reihe 4: ist leer

In den Reihen 6, 10, 12 und 15 zeigt die Schwärzung des Films die Gegenwart von Fremd-DNA an, die in das Genom von Dactylis glomerata L. Zellen integriert ist. Das 1063 Bp umfassende Fragment, das sich bei einer Verdauung des integrierten Hygromycingens von Plasmid pCIB709 mit BamHl erwarten lässt (Nukleotide 583-1646 von pCIB709), ist mit einem Pfeil markiert.

### Definitionen

Um ein klares und eindeutiges Verständnis der Beschreibung und der Ansprüche sowie der darin verwendeten Begriffe zu gewährleisten, werden die folgenden Definitionen aufgestellt:
Pflanzenzelle: Die strukturelle und physiologische Einheit einer Pflanze, die aus einem Protoplasten und einer Zellwand besteht.
Pflanzengewebe: Eine Gruppe von Pflanzenzellen, die in Form einer strukturellen und funktionellen Einheit organisiert ist.
Pflanzenorgan: Ein abgegrenzter und sichtbar differenzierter Bereich einer Pflanze, wie z.B. Wurzel, Stengel, Blätter, Blütenknospen oder Embryonen. Ein Pflanzenorgan kann aus verschiedenen Zell- oder Gewebetypen aufgebaut sein.
Protoplast: Isolierte Pflanzenzelle ohne Zellwand
Zellkultur: Proliferierende Zellmasse in einem undifferenzierten oder teilweise differenzierten Zustand.
Embryo: Ein sehr kleines und frühes Entwicklungsstadium einer Pflanze, das sich entweder aus einer Zygote (sexueller Embryo) oder aus einer embryogenen somatischen Zelle (somatischer Embryo) entwickelt und bereits Stadien mit erkennbarer Morphologie, Struktur und zellulärere Organisation aufweist, die zelluläre, globuläre sowie Kotyledonar-Stadien umfassen. (Die Embryonalentwicklung von Mais ist beispielsweise bei Randolph, 1936 beschrieben; die Embryonalentwicklung von Gräsern bei Brown, 1960).
Zellkluster: Eine Gruppe untereinander verbundener Zellen, die aneinanderhaften; gewöhnlich gehen diese Zellkluster aus einer oder wenigen Vorgängerzellen oder -protoplasten durch Zellteilung hervor.
Jungpflanze: Eine multizelluläre Struktur, die aus einem Spross und einer Wurzel besteht und die äussere Form einer kleinen Pflanze aufweist.
Dicamba: 3,6-Dichlor-2-methoxybenzoesäure
MES: 2-[N-morpholin]ethansulfonsäure
2,4-D: 2,4-Dichlorphenoxyessigsäure
Picloram: 4-Amino-3,6,6-trichlorpicolinsäure
Tris-HCI: a,a,a-Tris(hydroxymethyl)methylaminohydrochlorid
EDTA: 1-Ethylendiamin-N,N,N',N'-tetraessigsäure
PEG: Polyethylenglykol
Agarose: Die Herstellung und Reinigung von Agarose ist z.B. bei Guiseley und Renn, (1975) beschrieben. Agarose ist einer der Bestandteile des Agar. Kommerziell erhältlicher Agar besteht normalerweise aus einer Mischung von neutraler Agarose und ionischem Agaropectin, das eine Vielzahl von Seitengruppen aufweist. Käufliche Agarose wird gewöhnlich mit Hilfe bekannter Verfahren aus Agar gewonnen. Dabei bleiben in der Regel eine Reihe der Seitenketten erhalten, die dann die physikochemischen Eigenschaften der Agarose, wie z.B. die Gelierungs- und Schmelztemperatur, bestimmen. Bei niedrigen Temperaturen schmelzende Agarose ('Low-melting Agarose'), insbesondere Sea Plaque^{®} Agarose ist besonders bevorzugt als Verfestigungsmittel im Rahmen des erfindungsgemässen Verfahrens.
SH-0 Medium: Hormon-freies Medium nach Schenk und Hildebrandt, (1972). (Das SH-Medium kann flüssig oder mit 0.8% (w/v) Agar oder mit 0.5% (w/v) GelRite^{®} verfestigt sein). Das Medium wird normalerweise mit Hilfe bekannter Massnahmen durch Hitze oder im Autoklaven bei einer Temperatur von 121 °C und einem Druck von 1,2 kg/cm² für ca. 15 bis 20 Minuten sterilisiert.
GelRite®: GelRite Gellan Gum, Scott Laboratories Inc., Fiskersville, RI, # 02823.
SH-30 Medium: SH-0 Medium mit 30 wm Dicamba
SH-45 Medium: SH-0 Medium mit 45 µm Dicamba
KM-8p Medium: 8p Medium nach Kao et al, (1975). Dieses Medium kann in flüssiger Form oder verfestigt mit Agar, Agarose oder GelRite^{®} vorliegen und kann ebensogut ohne Ascorbinsäure, Vitamin A und Vitamin D hergestellt und verwendet werden. Die Medienbestandteile, mit Ausnahme des Verfestigungsmittels, werden normalerweise über eine Filtration durch ein 0.2 pm Filter sterilisiert.
RY-2 Medium: Medium nach Yamada et al, (1986).
OMS Medium: Medium nach Murashige und Skoog, (1962). Das Medium kann beispielsweise durch 0.8% (w/v) Agar oder Agarose oder mit 0.5% (w/v) GelRite^{®} verfestigt werden. Für den in der vorliegenden Anmeldung beschriebenen Verwendungszweck kann dieses Medium auch in der Weise hergestellt werden, dass es die Vitaminzusammensetzung des B5 Mediums nach Gamborg et al, (1968) aufweist.
Cellulase RS: Cellulase RS, Yakult Honsha Co., Ltd., 1.1.19 Higashi-Shinbashi, Minato-ku, Tokyo 105, Japan.
Pectolyase Y-23®: Seishin Pharmaceutical Co., Ltd., 4-13 Koami-cho, Nihonbashi, Tokyo, Japan.
Parafilm^{®}: Parafilm" laboratory film - American Can Co., Greenwich, CT, 06830, USA.
Nalgene^{®}-Filter: Nalge Co., Division of Sybron Corp. Rochester, New York, 14602, USA.
Bglll: Restriktionsenzym Bglll; New England Biolabs, 32 Tozer Road, Beverly, MA, 01915, USA, oder jeder beliebige andere Hersteller.
BaMHI: Restriktionsenzym BamHl; New England Biolabs, 32 Tozer Road, Beverly, MA, 01915, USA, oder jeder beliebige andere Hersteller.
Caseinhydrolysat: Caseinhydrolysat - Enzymatisches Hydrolysat aus Rindermilch, Typ 1, Sigma Co., PO. Box 14508, St. Louis, MO 63178, USA.
Hgrommycin B: Cytotoxin: Hygromycin B, gereinigt; Kat. Nr. 400050 Calbiochem Behring Diagnostica, La Jolla, CA 92037, USA, Chargen Nr. 702296.
GeneScreen Plus⁹: Kat. Nr. NEF 976, NEN Research Products, 549 Albany St., Boston, MA 02118, USA.
TBE Puffer: Tris-Boratpuffer - gebräuchlicher Puffer für die Elektrophorese, siehe Maniatis et al, (1982).
Spin Säule: Sephadex® G25 Fertigpack-Säule, Kat. Nr. 100402, Boehringer Mannheim Biochemicals, Piscataway, NJ, USA.
SDS: Sodiumdodecylsulfat.
SSC: 1.54 mM NaCl, 0.154 mM Natriumcitrat, gemäss der Beschreibung bei Maniatis et al, (1982).
CETAB: Hexadecyltrimethylammoniumbromid.
IBI Random Primer Kit: 'Prime Time' Random Primer Kit, International Biotechnologies Inc., PO. Box 1565, New Haven, CT 07606, USA. (Kat. Nr. 77800; Chargen Nr. F630-01)

Im Rahmen dieser Erfindung konnte jetzt überraschenderweise gezeigt werden, dass fertile Gramineen-Pflanzen, aus der Unterfamilie der Pooideae ausgehend von einem spezifischen Typ von Protoplasten sowie von Zellen und Kalli, die aus diesen Protoplasten hervorgehen, regeneriert werden können.

Diese Regeneration von fertilen Pflanzen, ist auch dann möglich, wenn die Protoplasten exogene DNA enthalten. Dabei handelt es sich vorzugsweise um stabil ins Genom der Protoplasten eingebaute DNA, die in Pflanzen exprimierbar ist.

Jede Gramineen-Pflanze aus der Unterfamilie Pooideae kann im Rahmen dieser Erfindung verwendet werden. Bevorzugt sind jedoch Pflanzen aus der Unterfamilie Pooideae, die zu den Gräsern gehören, so z.B. jede Gattung ausgewählt aus der Gruppe bestehend aus Poa, Festuca, Lolium, Bromus, Trisetum, Agrostis, Phleum, Alopecurus und Dactylis. Besonders bevorzugt sind Dactylis Pflanzen.

Ebenfalls bevorzugt im Rahmen dieser Erfindung sind Pflanzen aus der Unterfamilie Pooideae, die zur Gruppe der kleinkörnigen Getreide gehören, so z.B. jede Gattung ausgewählt aus der Gruppe bestehend aus Avena (Hafer), Triticum (Weizen), Secale (Roggen) und Hordeum (Gerste).

Der spezifische Typus von Pooideae Protoplasten, der sich teilt und Zellkulturen bildet, die in der Lage sind zu ganzen Pflanzen zu regenerieren, stammt aus Zellkulturen, inbesondere aber aus embryogenen Zellkulturen. Bei den embryogenen Zellkulturen handelt es sich vorzugsweise um embryogene Suspensions- oder Kalluskulturen. Die Zellkulturen können aus geeigneten Teilen von Pflanzen aus der Unterfamilie Pooideae hergestellt werden. Unter geeigneten Pflanzenteilen sind im Rahmen dieser Erfindung beispielsweise die basalen Abschnitte junger, innenliegender Blätter, unreife sexuelle Embryonen, unreife Blütenstände, reife Samen oder Keimlingsgewebe zu verstehen, die von Pflanzen aus der Unterfamilie Pooideae stammen, ohne jedoch darauf beschränkt zu sein.

### Verfahrensschritt A: Herstellung embryogener Suspensionen aus Pflanzengewebe

Zur Herstellung eines embryogenen Kallus geht man von einem geeigneten Abschnitt einer Pflanze aus der Unterfamilie Pooideae aus, in der Regel vom basalen Abschnitt eines jungen Blattes. Besonders bevorzugt sind die jüngeren, innen gelegenen Blätter von Pflanzen aus der Unterfamilie Pooideae. Dieser Verfahrensschritt kann analog der Beschreibung von Hanning et al, (1982) für Dactylis glomerata L. durchgeführt werden. Das dort beschriebene Verfahren ist nicht auf diese Spezies beschränkt, sondern kann auch für alle übrigen Pflanzen aus der Unterfamilie Pooideae Anwendung finden. Diese Publikation ist in Form einer Referenz ein Bestandteil der vorliegenden Erfindung.

Die Blätter werden beispielsweise in kleine Abschnitte oder Segmente von 1 mm bis 5 mm Länge oder Durchmesser zerschnitten. Die Form und Grösse dieser Blattstücke ist nicht kritisch. Diese Segmente werden auf einem für die Kallusinduktion und - erhaltung geeigneten Medium ausplattiert und dort bis zur Ausbildung eines Kallus und/oder embryogener Strukturen kultiviert. Ein für diesen Zweck geeignetes Medium ist z. B. ein SH-Medium (Schenk und Hildebrandt, 1972) mit 30 µM Dicamba und 0.8% (w/v) Agar oder Agarose als Gelierungsmittel. Weitere geeignete Medien sind bei George et al, (1987) beschreibenen. Kallus und/oder embryogene Strukturen erscheinen in der Regel 2 bis 6 Wochen nach dem Ausplattieren. Die Initiierung und Erhaltung des Kallus kann im Licht oder aber vorzugsweise im Dunkeln, bei einer Temperatur zwischen 0°C und 50°C, vorzugsweise aber bei einer Temperatur zwischen 20°C und 32°C und ganz besonders bevorzugt bei einer Temperatur zwischen 25°C und 28°C, erfolgen. Embryogener Kallus kann auch mit Hilfe anderer bekannter Verfahren hergestellt werden, die z.B. bei Lührs und Lörz, (1987) sowie in den darin enthaltenen Referenzen für Gerste beschrieben sind. Diese Verfahren können auch auf andere Pooideae Anwendung finden und bilden entsprechend in Form einer Referenz einen Bestandteil dieser Erfindung.

Die Etablierung embryogener Suspensionskulturen beginnt damit, dass man frische Stücke des embryogenen Kallus in ein geeignetes flüssiges Medium gibt, z.B. 0.5 g Kallus in 50 ml eines flüssigen Mediums nach Gray et al, (1985), das 45 µM Dicamba und 4 g/Liter Caseinhydrolysat enthält. Weitere geeignete Medien sind beispielsweise bei George et al, (1987) beschrieben. Die Suspensionskulturen wachsen bei einer Temperatur zwischen 10°C und 40°C, vorzugsweise zwischen 20°C und 32°C und ganz besonders bevorzugt zwischen 25°C und 30°C. Ein Wechsel zwischen Hell-und Dunkelphasen während der Kultivierungsperiode kann von Vorteil sein. Die Suspension wird vorzugsweise mit einer 5 bis 20 stündigen, insbesondere aber einer 16 stündigen Lichtperiode kultiviert, gefolgt von einer 5 bis 20 stündigen, vorzugsweise aber einer 8 stündigen Dunkelperiode. Die Lichtintensität liegt typischerweise zwischen 0.1 µE/m²s und 100 µE/m²s (E=Einstein; m=Meter; s=Sekunde), vorzugsweise aber zwischen 30 µE/m²s und 80 µE/m²s. Es ist ebenso vorteilhaft, die Suspension während der Kultivierungsphase zu bewegen. Die Bewegung der Suspension kann beispielsweise in Delong Flaschen, die mit einem für Licht und Gas durchlässigen Plastikfilm oder einem beliebigen anderen geeigneten Verschluss abgedichtet sind, auf einer Rundschüttelmaschine bei einer Umdrehungsgeschwindigkeit von 100 Upm bis 150 Upm durchgeführt werden. Nach ca. drei bis fünf Wochen lässt man die grösseren Zell-Zusammenballungen für ca. 30 Sekunden absetzten, entfernt den Überstand, der nur Weine Zellkluster enthält und überträgt diesen zur Initiation neuer Kulturen in ein frisches Medium.

Diese Verfahrensschritte können periodisch, vorzugsweise alle 3 bis 4 Wochen, wiederholt werden, wobei jeweils nur die erfolgversprechensten Kulturen verwendet werden, gemessen an der geringen Grösse sowie der Qualität der Zellldumpen. Nach 4 bis 20, in der Regel jedoch nach 6 bis 8 Übertragungen (Transfers) sind die Suspensionen im wesentlichen frei von nicht-embryogenen Zellen und die Mehrzahl der embryogenen Zellkluster weist typischerweise eine Grösse von 150 µm bis 2'000 µm auf.

Besonders bevorzugt im Rahmen dieser Erfindung ist ein Verfahren, das zur Herstellung embryogener Suspensionen führt, die in erster Linie kleine präembryogene Zellmassen aufweisen. Der Anteil der präembryogenen Zellmassen kann noch dadurch in signifikanter Weise gesteigert werden, dass man zunächst das grössere Material absetzten lässt und anschliessend nur den oben befindlichen Teil der Suspension erneut kultiviert.

Somit betrifft ein Gegenstand der vorliegenden Erfindung eine embryogene Zellkultur, Suspensionskultur oder Kalluskultur, die sich von Gramineen-Pflanzen aus der Unterfamilie Pooideae ableitet und aus der Protoplasten isoliert werden können, wobei besagte Protoplasten in der Lage sind Zellwände zu regenerieren, sich zu teilen und Kallus auszubilden. Besagter Kallus kann dann seinerseits zu ganzen Pflanzen, insbesondere aber zu ganzen fertilen Pflanzen regeneriert werden.

Besonders bevorzugt im Rahmen der vorliegenden Erfindung sind embryogene Zellkulturen (Suspensionskulturen und Kalluskulturen) von Gräsern, insbesondere von Gräsern ausgewählt aus den Gattungen bestehend aus Poa, Festuca, Lolium, Bromus, Trisetum, Agrostis, Phleum, Alopecurus und Dactylis. Ganz besonders bevorzugt sind embryogene Zellkulturen von Dactylis glomerata L..

Weitere im Rahmen der vorliegenden Erfindung bevorzugte Gegenstände betreffen embryogene Zellkulturen (Suspensions- und Kalluskulturen) von kleinkörnigen Getreiden, insbesondere von Getreiden ausgewählt aus den Gattungen bestehend aus Avena, Triticum, Secale und Hordeum.

Ein weiterer Gegenstand der vorliegenden Erfindung betrifft den embryogenen Kallus, der sich von Gramineen-Pflanzen aus der Unterfamilie Pooideae ableitet und von dem Protoplasten isoliert werden können, wobei besagte Protoplasten in der Lage sind Zellwände zu regenerieren, sich zu teilen und Kallus auszubilden, der dann seinerseits wieder zu ganzen Pflanzen, insbesondere aber zu ganzen fertilen Pflanzen regeneriert werden kann.

Besonders bevorzugt im Rahmen der vorliegenden Erfindung ist ein embryogener Kallus von Gräsern, insbesondere von Gräsern ausgewählt aus den Gattungen bestehend aus Poa, Festuca, Lolium, Bromus, Trisetum, Agrostis, Phleum, Alopecurus und Dactylis. Ganz besonders bevorzugt ist ein embryogener Kallus von Dactylis glomerata L..

Ein weiterer, im Rahmen der vorliegenden Erfindung bevorzugter Gegenstand betrifft einen embryogenen Kallus, der sich von kleinkörnigen Getreiden ableitet, insbesondere von Getreiden ausgewählt aus den Gattungen bestehend aus Avena, Triticum, Secale und Hordeum.

Ebenfalls umfasst von der vorliegenden Erfindung sind Gramineen-Pflanzen, insbesondere aber fertile Gramineen-Pflanzen, aus der Unterfamilie Pooideae und deren Vermehrungsgut, die ausgehend von Protoplasten oder pflanzlichen Zellen, welche aus einer embryogenen Zellkultur stammen, regeneriert werden.

### Verfahrensschritt B: Isolierung und Reinigung von Protoplasten, die zu ganzen Pflanzen, einschliesslich ganzen fertilen Pflanzen regeniert werden können

Ausgehend von den aus Verfahrensschritt A) erhältlichen embryogenen Suspensionskulturen werden Protoplasten gewonnen. Die Isolierung und Reinigung der Protoplasten erfolgt in der Weise, dass zunächst embryogene Zellkluster aus dem Suspensionskulturmedium isoliert werden, z.B. durch Filtration einer Suspensionskultur aus Verfahrensschritt A) über eine Nalgene⁰ Filtereinheit (0.2 µm) und die resultierenden Zellkluster anschliessend mit einer geeigneten Enzymlösung inkubiert werden, die in der Lage ist die Zellwände ohne Schädigung der Protoplasten zu entfernen. Die Enzyme werden in Form einer Filter-sterilisierten Lösung verwendet. Alle Manipulationen, die in Zusammenhang mit Zell- oder anderen Kulturen stehen, werden unter sterilen Bedingungen und unter Verwendung von sterilen Materialien durchgeführt. Eine geeignete Enzymlösung kann beispielsweise die folgende Zusammensetzung aufweisen:

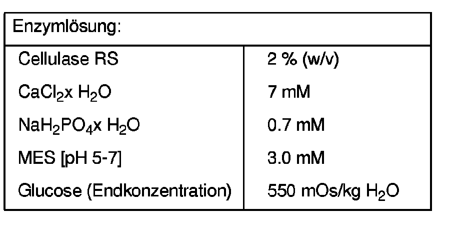

Normalerweise wird diese Mischung auf einer Schüttelmaschine bei einer Umdrehungsgeschwindigkeit von etwa 50 Upm und schwacher Beleuchtung (ca. 5 wE/m2s) vorsichtig bewegt, ohne dass dies kritisch wäre. Die Verdauung wird bei einer Temperatur zwischen 0°C und 50°C, vorzugsweise zwischen 10°C und 35°C und ganz besonders bevorzugt zwischen 26°C und 32°C fortgesetzt, bis die Protoplasten freigesetzt werden. Die für die Verdauung benötigte Zeit beträgt typischerweise zwischen einigen Sekunden und 2 Tagen, vorzugsweise zwischen 1 Stunde und 1 Tag und ganz besonders bevorzugt zwischen 3 und 5 Stunden.

Die freigesetzten Protoplasten werden mit Hilfe von Standardverfahren wie z.B. Filtration, Zentrifugation und Spühlen gesammelt und gereinigt.

An dieser Stelle kann ein weiterer Reinigunsschritt eingefügt werden. Dabei werden die Protoplasten auf die Oberfläche eines geeigneten Mediums, wie z.B. eines KM-8p Kulturmediums, das mit Saccharose auf einen osmotischen Wert von 700 mOs/kg H₂0 eingestellt wird, oder anderer geeigneter Medien, die z.B. bei George et al, (1987) beschrieben sind, aufgebracht.

Nach zehnminütiger Zentrifugation bei etwa 60 g werden die Protoplasten, die sich im Bereich der Grenzfläche befinden, gesammelt. Anschliessend können die Protoplasten im gleichen Kulturmedium resuspendiert und beispielsweise durch Passieren eines Stahlsiebes (Maschenweite 20 µm) filtriert werden.

Ohne diesen zusätzlichen Reinigungsschritt liegt die Kontamination der Protoplastenzubereitung mit ganzen, nicht verdauten Zellen in einem Bereich von etwa 0.001% bis 0.01% der Gesamtzellzahl. Dieser Wert kann durch den oben beschriebenen Reinigungsschrittes noch weiter reduziert werden. Dieser zusätzliche Verfahrensschritt führt jedoch zu einem signifikanten Verlust an Protoplasten, welche ein sehr dichtes Cytoplasma aufweisen. Daraus resultiert, dass die Plattierungseffizienz bis zum zehnfachen niedriger liegen kann, wenn der besagte Reinigungsschritt eingeschlossen ist. Die Protoplasten können somit auch mit Hilfe anderer bekannter Verfahren gereinigt werden, wie z.B. durch die oben beschriebene Flotation auf einer Saccharoselösung oder auf anderen geeigneten Pufferlösungen mit hoher Dichte, wie z.B. einer Percoll^{®} Lösung.

Die Protoplastenausbeute und in der Folge die Plattierungseffizienz erreichen optimale Werte, wenn von den Suspensionskulturen, die für die Protoplastenisolierung verwendet werden, 1 bis 30 Tage, vorzugsweise aber 5 bis 10 Tage vor der Protoplastenisolierung Folgekulturen angelegt werden.

Bei der oben näher bezeichneten Enzymlösung handelt es sich um eine Modifikation des bei Lu et al, (1981) beschriebenen Enzymgemisches, die allen anderen getesteten Enzymlösungen überlegen ist und einen Ertrag von 40 x 10⁶ bis 70 x 10⁶ Protoplasten pro Gramm Frischgewicht ergibt. Alternativ dazu lassen sich jedoch auch bei Verwendung von 2% (w/v) Cellulose RS im KM-8p Medium oder in einem beliebigen anderen Medium, wie sie beispielsweise bei George et al, (1987) beschrieben sind, beachtliche Protoplastenausbeuten erzielen. Dabei erweist sich die Verwendung von Glucose als Osmoticum bei der Protoplastenisolierung der Saccharose eindeutig und dem Mannitol in gewissem

Umfang überlegen, was die Ausbeute und in der Folge die Plattierungseffizienz angeht. Weitere bekannte und geeignete Enzymmischungen können in dem erfindungsgemässen Verfahren ebenfalls Anwendung finden.

Die nach der Filtration, d.h. z.B. nach Passieren eines 20 pm Siebes mit einer durchschnittlichen Maschenweite von 12 µm bis 15 µm im Durchmesser, erhältlichen Protoplasten weisen ein optisch dichtes Cytoplasma auf.

Die vorliegende Erfindung betrifft somit ein Verfahren zur Herstellung von Protoplasten, die zu ganzen Pflanzen, insbesondere aber zu ganzen fertilen Pflanzen regeneriert werden können, ausgehend von Gramineen- Pflanzen der Unterfamilie Pooideae. Dieses Verfahren ist durch die folgenden Verfahrensschritte gekennzeichnet:
(a) Isolierung von Gewebe aus geeigneten Teilen von Gramineen-Pflanzen der Unterfamilie Pooideae, vorzugsweise aus den basalen Abschnitten junger, innen liegender Blätter, aus unreifen sexuellen Embryoen, unreifen Blütenständen, reifen Samen oder Keimlingsgeweben, ganz besonders bevorzugt aber aus den jüngsten und am weitesten innen liegenden Blättern;
(b) Kultivierung dieses Gewebes in einem Medium, das in der Lage ist die Ausbildung von embryogenem Kallus und von Embryonen zu induzieren;
(c) Periodisches Anlegen von Folgekulturen des embryogenen Kallus und der Embryonen auf frischem Medium, indem man kleine Zellkluster mit cytoplasmareichen Zellen in frischem Medium überträgt, das eine kontinuierliche Proliferation gewährleistet;
(d) Isolierung embryogener Zellkluster, mit einer Grösse von 150-2000 wm, nach 0 bis 500, vorzugsweise nach 0 bis 100 und ganz besonders bevorzugt nach 3 bis 50 Übertragungen (Transfers); und
(e) Entfernen der Zellwände mit geeigneten Enzymmischungen und Isolierung und Reinigung der resultierenden Protoplasten.

Ein weiterer Gegenstand dieser Erfindung umfasst Protoplasten (einschliesslich der nach Regeneration der Zellwände erhältlichen pflanzlichen Zellen) von Gramineen-Pflanzen der Unterfamilie Pooideae, die zu ganzen Pflanzen und insbesondere zu ganzen fertilen Pflanzen regeneriert werden können. Bevorzugt sind Protoplasten oder Zellen, die entweder aus Zellkulturen oder aus embryogenen Zellsuspensionen gewonnen werden.

Ebenso umfasst sind Gramineen Pflanzen, insbesondere aber fertile Gramineen Pflanzen, aus der Unterfamilie Pooideae und deren Vermehrungsgut, die aus besagten Protoplasten oder aus gesagten Pflanzenzellen regeneriert werden.

### Verfahrensschritt C: Errichtung von Protoplastenkulturen und Heranziehen von Kallus. der zu ganzen Pflanzen und zu ganzen fertilen Pflanzen regeniert werden kann

Die gereinigten Protoplasten aus Verfahrensschritt B) werden in einem geeigneten Flüssigmedium oder auf einem geeigneten Festmedium ausplattiert, unabhängig davon, ob besagte Protoplasten zuvor mit exogener DNA behandelt wurden oder nicht. (Die Behandlung mit exogener DNA wird in einem der folgenden Abschnitte im Einzelnen beschrieben.) Unter geeigneten Medien sind im Rahmen dieser Erfindung beispielsweise solche Medien zu verstehen, die auf einem KM-8p-, einem RY-2- (Potrykus et al, 1979), einem SH-30- oder einem SH-45-Medium basieren und die eine geeignete Konzentration an Zuckern und pflanzlichen Wachstumsregulatoren aufweisen. Die bevorzugten Medien sind das KM-8p- und das SH-45-Medium, die ein geeignetes Verfestigungsmittel enthalten. Das bevorzugte Verfestigungsmittel ist Agarose, insbesondere SeaPlaque^{®} Agarose (FMC Corp. Marine Colloids Division, PO. Box, Rockland, ME 04841, USA). Bei Verwendung von SeaPlaque^{®} Agarose kann deren Konzentration zwischen 0.1% und 2.5% (w/v). vorzugsweise aber zwischen 0.6% und 1.5% (w/v) betragen.

Das Ausplattieren der Protoplasten auf einem Agarose-haltigen Medium kann analog zu den bei Shillito et al, (1983), in der Europäischen Patentanmeldung EP-0,129,688 (Shillito et al oder bei Adams etal, (1983) beschriebenen Verfahren durchgeführt werden. Diese Publikationen sind in Form einer Referenz Bestandteil dieser Erfindung.

Das Medium, in dem die Protoplasten kultiviert werden, kann weitere geeignete Bestandteile enthalten, welche die Teilung und Koloniebildung der Protoplasten unterstützen. Zu diesen Substanzen gehören beispielsweise 2,4-D, Dicamba, Picloram oder andere Pflanzenwachstumsregulatoren. Geeignete Pflanzenwachstumsregulatoren sind dem Fachmann auf diesem Gebiet bekannt. Die Konzentration dieser Substanzen liegt üblicherweise in einem Bereich von 0.01 mg/Liter bis 100 mg/Liter.

Salicylsäure und dessen Derivate können die Zellteilung und Koloniebildung der Pooideae Protoplasten fördern. Zu den Derivaten der Salicylsäure gehören, ohne jedoch darauf beschränkt zu sein, die O-Acyl und O-Aryl Derivate. Zu den O-Acyl Derivaten zählen, ohne darauf beschränkt zu sein, Niederacyl-Gruppen mit beispielsweise 1 bis 7, vorzugsweise mit 1 bis 4 und ganz besonders bevorzugt mit 2 bis 3 Kohlenstoffatomen, Unter O-Aryl Derivaten sollen im Rahmen der vorliegenden Erfindung 5- oder 6-gliedrige Ringe verstanden werden, die miteinander fusioniert oder aber nicht fusioniert sein können, ohne jedoch darauf beschränkt zu sein. Die Ringe können unsubstituiert oder mit einer oder mehreren Gruppen substituiert sein, einschliesslich Alkyl mit 1 bis 5 Kohlenstoffatomen, O-Alkyl mit 1 bis 4 Kohlenstoffatomen, Halogen (insbesondere Chlorid und Bromid), Nitro, Amino und Amino, das seinerseits substituiert ist mit Alkyl, das 1 bis 4 Kohlenstoffatome aufweist.

Die Derivate der Salicylsäure beinhalten auch die Carboxylatester. Bevorzugte Carboxylatester sind die Aryl- und Alkylester, worin die Alkylgruppe 1 bis 4 Kohlenstoffatome umfasst.

Darüberhinaus sind under Derivaten der Salicylsäure auch solche Verbindungen zu verstehen, bei denen der Salicylsäurering noch weiter substituiert ist, beispielsweise durch eine oder mehrere Gruppen einschliesslich Alkyl mit 1 bis 4 Kohlenstoffatomen, O-Alkyl mit 1 bis 4 Kohlenstoffatomen, Halogen (insbesondere Chlorid und Bromid), Nitro, Amino und Amino, das seinerseits substituiert ist mit Alkyl, das 1 bis 4 Kohlenstoffatome aufweist.

Bevorzugte Verbindungen, die die Teilung und/oder Kolonierbildung der Pooideae Protoplasten und Zellen fördern sind:
O-Acetoxybenzoesäure (Aspirin, Acetylsalicylsäure);
O-Hydroxybenzoesäure (Salicylsäure);
O-Methoxybenzoesäure (Methylsalicylsäure); und
O-Dimethylcarbamoylbenzoesäure [O-(CO-dimethyl)-salicylsäure].

Die Konzentration der Salicylsäure oder einer ihrer Derivate im Kulturmedium liegt vorzugsweise in einem Bereich von 0.1 mg/Liter und 3'000 mg/Liter, vorzugsweise von 10 mg/Liter und 300 mg/Liter und ganz besonders bevorzugt bei etwa 100 mg/Liter Das Medium, in welchem die Protoplasten kultiviert werden, kann zusätzlich noch Medium enthalten, das zuvor durch das Wachstum geeigneter Zellen, die z.B. von Zea mays, Dactylis glomerata oder von anderen Gramineen oder auch von anderen (dikotyledonen) Pflanzen stammem, konditioniert wurde. Bevorzugt ist ein Medium, in welchem eine embryogene Suspension einer Gramineenart kultiviert wurde. Ganz besonders bevorzugt ist ein Medium, in welchem eine embryogene Suspension von Dactylis glomerata kultiviert wurde. Das auf die zuvor beschriebene Weise konditionierte Medium kann einen Anteil zwischen 0% und 100% (v/v), vorzugsweise zwischen 5% und 50% (v/v) und ganz besonders bevorzugt zwischen 30% und 40% (v/v) am Gesamtmedium ausmachen.

Die Protoplasten können in einem festen oder in einem flüssigen Medium über einen Zeitraum von bis zu 12 Wochen, vorzugsweise von bis zu 6 Wochen und ganz besonders bevorzugt von 1 bis 3 Wochen, kultiviert werden, ohne das zwischenzeitlich Folgekulturen angelegt werden. In einer besonderen Ausführungsform der vorliegenden Erfindung kann ein Festmedium in ein flüssiges Medium eingebracht werden, wie dies in der Europäischen Patentanmeldung EP-0,129,688 (Shillito et a0 beschrieben ist, oder aber es kann auf irgendeine andere Weise behandelt werden, die die Zellteilung und/oder Koloniebildung der Protoplasten unterstützt.

Die Protoplasten werden im Licht oder aber vorzugsweise im Dunkeln bei einer Temperatur zwischen 0°C und 50°C, vorzugsweise zwischen 20°C und 32°C und ganz besonders bevorzugt zwischen 25°C und 28°C, kultiviert. Die Lichtintensität beträgt typischerweise zwischen 0.1 wE/m²s und 200 µE/m²s, vorzugsweise aber zwischen 30 µE/m²s und 90 wE/m2s.

Die Plattierungseffizienz bei Verwendung eines KM-8p-Mediums schwankt zwischen 0.5% und 10%, abhängig von der Qualität der Protoplastenpräparation. Die Zugabe von 30% bis 40% (v/v) eines konditionierten Suspensionskulturmediums (Suspensionskulturmedium, konditioniert durch das Wachstum der darin befindlichen Zellen und eingestellt auf einen osmotischen Wert von 550 mOsm/kg H₂0 durch Zugabe von Glucose) zum Protoplastenkulturmedium führt zwar nicht zu einem signifikanten Anstieg der Plattierungseffizienz, beschleunigt aber den Teilungsprozess in den jungen, aus den Protoplasten hervorgegangenen Kolonien.

In einer bevorzugten Ausführungsform der vorliegenden Erfindung werden die Protoplasten auf einem mit Agarose verfestigten Medium ausplattiert. Die ersten Zellteilungen lassen sich dann ca. zwei Tage nach dem Ausplattieren der Protoplasten beobachten. Weitere Teilungen erfolgen alle 2 bis 3 Tage. Dieser Teilungsprozess verläuft nicht synchronisiert, was sich durch die Tatsache belegen lässt, dass erste Zellteilungen auch erst nach 7 Tagen auftreten können. 5 bis 20 Tage, vorzugsweise aber 10 bis 14 Tage nach dem Ausplattieren, wird das mit Agarose verfestigte Medium in Segmente zerschnitten und die Segmente, welche die Zellkolonien beinhalten, werden in ein flüssiges Nährmedium transferiert. Dieses Verfahren ist unter der Bezeichnung 'bead culture technique' allgemein bekannt und ist bei Shillito et al, (1983) sowie in der Europäischen Patentanmeldung EP-0,129,688 (Shillito et a4 vollständig beschrieben.

Anstatt das mit Agarose verfestigte Medium zu segmentieren, kann dieses auch verflüssigt und in dieser flüssigen Form in das flüssige Nährmedium überführt werden. Diese Modifikation ist bei Adams et al, (1983) beschrieben und kann in analoger Weise durchgeführt werden. In beiden Fällen (Segmentieren oder Verflüssigen) kann KM-8p-Medium mit Glucose und Saccharose als flüssige Medienkomponente verwendet werden, wobei ein gutes Koloniewachstum zu beobachten ist. Die optimale Flüssigkomponente im Hinblick auf das Koloniewachstum besteht jedoch aus einem SH-45-Medium mit 4 g/Liter Caseinhydrolysat. Innerhalb von etwa 2 bis 3 Wochen seit dem Ansetzen der 'bead' Kulturen lassen sich innerhalb der Platten neue Suspensionskulturen erkennen. Die mikroskopische Untersuchung der Agarosescheiben ergibt, dass normalerweise einige der am weitesten an der Oberfläche gelegenen Kolonien in das Flüssigmedium herauswachsen und kleinere Zellmengen in das Medium freisetzen, während der Rest der Kolonie weiterhin fest in der Agarose verankert bleibt. Die neue Suspension vermehrt sich sehr rasch und nach zwei weiteren Wochen wird diese in gleicher Weise wie normale Suspensionskulturen transferiert oder aber auf SH-30 Medien zur Entwicklung von Kallus ausplattiert. In einer alternativen Ausführungsform kann die Agarose auch auf Petrischalen, die ein mit Agarose verfestigtes SH-45 Medium enthalten, verteilt und anschliessend kultiviert werden.

Die vorliegende Erfindung betrifft somit auch ein Verfahren zur Herstellung von Zellkulturen (Suspensions- und Kalluskulturen) aus Protoplasten von Gramineen-Pflanzen der Unterfamilie Pooideae, die zu ganzen Pflanzen, insbesondere aber zu ganzen fertilen Pflanzen regeneriert werden können. Dieses Verfahren ist durch die folgenden Verfahrensschritte gekennzeichnet:
(a) Kultivierung von Protoplasten, die von Gramineen-Pflanzen aus der Unterfamilie Pooideae stammen und die zu ganzen Pflanzen regeneriert werden können, in einem geeigneten Kulturmedium bis zur Ausbildung von Zellkolonien;
(b) Kultivierung besagter Zellkolonien oder von Teilen davon auf einem geeigneten Medium, da die Ausbildung von Zellkulturen fördert; und
(c) Isolierung der resultierenden Zellkulturen.

Verfahrensschritt (b) ist nicht unbedingt notwendig. Es ist in gleicher Weise möglich, die Protoplasten in dem in Verfahrensschritt (a) definierten Medium zu belassen, bis sich Zellkulturen oder Embryonen ausbilden.

Ebenfalls umfasst sind Gramineen-Pflanzen, insbesondere aber fertile Gramineen-Pflanzen, der Unterfamilie Pooideae und deren Vermehrungsgut, die aus besagten Zellkulturen regeneriert werden.

Ein bevorzugter Gegenstand der vorliegenden Erfindung betrifft das Ausplattieren von Protoplasten auf einem mit Agarose verfestigten Medium, Verflüssigen oder Segmentieren des mit Agarose verfestigten Mediums, Transferieren des verflüssigten oder segmentierten Mediums in ein flüssiges Nährmedium und Kultivierung bis zur Ausbildung von Zellkolonien.

Bevorzugt ist ein Verfahren worin die unter Verfahrensschritt (b) erwähnten Teile der Zellkolonien von Zellen oder Zellmassen abstammen, die in das flüssige Kulturmedium freigesetzt wurden.

Die in diesem und in anderen Verfahrensschritten hergestellten Kallus-und Suspensionskulturen können analog zu der Beschreibung in Verfahrensschritt A) in der Kälte konserviert werden.

### Verfahrensschritt D: Regeneration von Junapflanzen aus Kallus

Von Kallus, der aus Protoplasten gewonnen wurde [Verfahrensschritt C], insbesondere wenn es sich um krümeligen, granulären Kallus handelt, werden ein oder mehrmals, vorzugsweise alle zwei Wochen, Folgekulturen auf einem frischen, geeigneten Medium angelegt, um auf diese Weise eine Embryobildung zu induzieren. Ein geeignetes induzierendes Medium ist beispielsweise ein SH-Medium, mit geeigneten Konzentrationen an Zuckern und Pflanzenwuchsregulatoren.

Alle auf diese Weise gebildeten Embryonen werden anschliessend auf ein Medium übertragen, welches geeigente Voraussetzungen für die Induktion der Reifung und Keimung bietet. Geeignete Medien sind beispielsweise ein SH-30 oder ein OMS Medium, die in einer Weise modifiziert sind, dass sie geeignete Konzentrationen an Zuckern und Pflanzenwachstumsregulatoren aufweisen. Die Kultivierung der Platten erfolgt im Licht [10 /-LE/m2s bis 200 wE/m²s; kaltes Weisslicht von fluoreszierenden Lampen oder eine Mischung aus Tageslicht und dem Licht einer Gro.Lux° (Sylvania) Fluoreszenzleuchte. Selbstverständlich kann auch jede beliebige andere geeignete Fluoreszenzleuchte verwendet werden. 2 bis 5 Wochen nach dem Ausplattieren lassen sich die ersten Embryonen beobachten. In manchen Fällen können ein oder mehrere zusätzliche Transfers auf frisches Medium für die Ausreifung der Embryonen von Vorteil sein. Die Embryonen entwickeln sich weiter und bilden nach einer gewissen Zeit, typischerweise nach 1 Woche bis 6 Monaten, insbesondere aber nach 1 bis 3 Monaten, Jungpflänzchen aus.

In einer alternativen Ausführungsform werden von dem aus Protoplasten gewonnenen Kallus [Verfahrensschritt C], insbesondere wenn es sich um krümeligen, granulären Kallus handelt, ein oder mehrmals, vorzugsweise alle zwei Wochen, Folgekulturen auf einem frischen, geeigneten Medium angelegt, um auf diese Weise die Ausbildung und Reifung von Embryonen zu induzieren. Ein für diese Zwecke geeignetes Medium ist beispielsweise, ohne jedoch darauf beschränkt zu sein, ein OMS Medium, das eine geeignete Konzentration an Zuckern aber keine Pflanzenwachstumsregulatoren aufweist. Die Kultivierung der Platten erfolgt im Licht [10 µE/m²s bis 200 wE/m²s; kaltes Weisslicht von fluoreszierenden Lampen oder eine Mischung aus Trageslicht und dem Licht einer Gro-Lux^{©} (Sylvania) Fluoreszenzleuchte oder jeder beliebigen anderen geeigneten Fluoreszenzleuchte]. Die Embryonen entwickeln sich weiter und bilden nach einer gewissen Zeit, typischerweise nach 1 Woche bis 6 Monaten, insbesondere aber nach 1 bis 3 Monaten, Jungpflänzchen aus.

### Verfahrensschritt E: Gewinnung von fertilen Pflanzen aus Junapflänzchen

Die zuvor in Verfahrensschritt D) erhaltenen Jungpflänzchen werden auf ein geeignetes Medium, beispielsweise auf ein SH-0 Medium oder ein OMS Medium ohne Wachstumsregulatoren transferiert. Alternativ dazu kann auch ein Wachstumsregulator zugegeben werden, der die Ausbildung von Wurzeln oder Sprossen stimuliert. Geeignete Wachstumsregulatoren sind dem Fachmann auf diesem Gebiet bekannt. Die Jungpflänzchen werden solange auf diesem Medium kultiviert, bis sie Wurzeln ausbilden. Dabei ist es wichtig, dass man jeglichen Kallus von den Jungpflänzchen entfernt, da es sich gezeigt hat, dass dieser Kallus das Wachstum der Pflänzchen negativ beeinflusst. Um dies zu erreichen, kann man die Pflänzchen, beispielsweise im Verlauf der Transfers, mit destilliertem Wasser abspühlen. Kallus, der sich erst nachträglich bildet, muss in regelmässigen Abständen, vorzugsweise alle 3 bis 30 Tage und besonders bevorzugt alle 1 bis 2 Wochen, entfernt werden. Der Zeitraum, der für die Wurzelbildung benötigt wird, beträgt typischerweise etwa 1 bis 4 Wochen, insbesondere aber 2 Wochen. Pflänzchen, die ein gut ausgebildetes Wurzelsystem besitzen, können dann in Erde übertragen und ins Gewächshaus überführt werden, wo sie nach und nach aushärten. Als ausreichend wird im diesem Zusammenhang eine Wurzellänge im Bereich zwischen 1 cm und 10 cm, insbesondere aber zwischen 2 cm und 5 cm angesehen. Beim Spross liegt der entsprechende Bereich ebenfalls zwischen 1 cm und 10 cm und insbesondere zwischen 2 cm und 5 cm. Alternativ dazu können die Pflänzchen auch durch Anlegen immer neuer Folgekulturen für einen unbestimmten Zeitraum in-vitro weiterkultiviert werden, indem man die Schösslinge voneinander trennt und die Pflänzchen auf frisches Medium, z.B. SH-0 oder OMS Medium, überträgt.

Somit ist das erfindungsgemässe Verfahren zur Regeneration von ganzen fertilen Gramineen-Pflanzen aus der Unterfamilie Pooideae durch die Verfahrensschritte in Anspruch 22 charakterisiert:
Die Blütenbildung kann entweder nach dem bei Heide, (1987) beschriebenen Verfahren oder aber nach jedem beliebigen anderen Verfahren induziert werden, das den Anforderungen der jeweils verwendeten Spezies oder Varietät gerecht wird. Verfahren zur Blühinduktion bei Pooideae sind bekannt.

Der von diesen Pflanzen produzierte Samen kann durch angemessene Behandlung zur Keimung gebracht und entweder in Töpfe ausgesät oder aber sterilisiert und auf ein Murashige und Skoog Medium, das keine Wachstumsregulatoren (OMS Medium) aufweist und mit 0.8% Agar oder Agarose, GelRite^{®} oder irgend einem beliebigen anderen Geliermittel verfestigt ist, ausplattiert werden. Der Samen kann auch auf ein Medium ausgesät werden, das zwischen 10 µg/ml und 1 '000 gg/ml Hygromycin B enthält, mit dessen Hilfe die Vererbbarkeit einer Hygromycinresistenz nachgewiesen werden kann.

Das erfindungsgemässe Verfahren zur Regeneration von fertilen Gramineen-Pflanzen der Unterfamilie Pooideae aus Kallus beinhaltet somit die folgenden Verfahrensschritte:
(a) Kultivierung von Kallus, der sich von Pflanzen aus der Unterfamlie Pooideae ableitet, der aus Protoplasten gewonnen wird gemäß Anspruch 6 und der zu ganzen Pflanzen regeneriert werden kann, auf einem Medium, das die Ausbildung von Embryonen zu induzieren vermag, bis zur Bildung von Embryonen;
(b) Kultivierung der Embryonen auf einem Medium, das geeignet ist, die Reifung und Keimung besagter Embryonen zu induzieren;
(c) Kultivierung der entstehenden Pflänzchen bis zu einem Stadium, in welchem diese in Erde übertragen und zu ganzen Pflanzen ausgereift werden können; und
(d) Gewinnung von Samen in Folge einer kontrollierten oder offenen Bestäubung.

En weiterer Gegenstand dieser Erfindung betrifft Gramineen-Pflanzen, insbesondere aber fertile Gramineen-Pflanzen, aus der Unterfamilie Pooideae und deren Vermehrungsgut, die aus besagtem Kallus regeneriert werden.

### Verfahrensschritt F: Behandlung von Protoplasten mit exogener DNA

Pooideae Protoplasten können mit exogener DNA behandelt werden, wobei Zellen entstehen, welche die gesamte oder aber einen Teil der exogenen DNA stabil in ihr Genom integriert enthalten. Unter exogener DNA soll im Rahmen dieser Erfindung jede DNA verstanden werden, die zu den Protoplasten hinzugefügt wird. Die besagte DNA kann entweder homolog oder aber heterolog sein in Bezug auf die transformierte Pflanze. Die exogene DNA kann eine Promoter enthalten, der in Pflanzen aus der Familie Gramineae, insbesondere aber in Pflanzen aus der Unterfamilie Pooideae, aktiv ist, oder aber man verwendet einen Promotor, der bereits im Genom der Pflanze vorliegt. Die exogene DNA kann darüberhinaus ein oder mehrere Gene enthalten, die den Genotyp, insbesondere aber den Phänotyp, der resultierenden Zelle oder der daraus regenerierten Pflanze verändern. Es ist jedoch wünschenswert, das die Gensequenz, die ein oder aber mehrere gewünschte proteinartige Produkte kodiert, exprimiert wird und ein oder mehrere funktionelle Enzyme oder Polypeptide in der resultierenden Zelle bzw. Pflanze produziert werden. Bei der exogenen DNA kann es sich um ein chimäres Gen oder aber um einen Teil davon handeln.

Unter exogener DNA soll im Rahmen dieser Erfindung definitionsgemäss auch nicht-kodierende DNA mit regulatorischer Funktion verstanden werden, die beispielsweise bei der Regulation der Transkription eine Rolle spielt.

Die Behandlung der Protoplasten mit exogener DNA kann nach einem der in den folgenden Publikationen beschriebenen Verfahren durchgeführt werden:
[Paszkowski et al, 1984; Europäische Patentanmeldung EP-0,164,575 (Paszkowski et a0; Shillito et al, 1985; Potrykus et al, 1985; Loerz et al, 1985; Fromm et al, 1986; Britische Patentanmeldung GB-2,140,822 (Mettler) und Negrutiu et al, 1987]. Diese Publikationen sind in Form einer Referenz ein Bestandteil dieser Erfindung.

Die exogene DNA kann in jeder beliebigen Form, beispielsweise als nackte lineare oder zirkuläre DNA, enkapsuliert in Liposomen, in Sphäroplasten, als Bestandteil anderer Protoplasten, komplexiert mit Salzen, etc., zu den Protoplasten zugegeben werden. Die Aufnahme der Fremd-DNA kann durch jedes beliebige geeignete und bekannte Verfahren, einschliesslich derjenigen, die in den zuvor aufgelisteten Publikationen beschrieben sind, stimuliert werden.

Bevorzugt im Rahmen dieser Erfindung sind chimäre Gene, die den transformierten Protoplasten sowie den daraus sich entwickelnden Geweben und insbesondere den Pflanzen vorteilhafte Eigenschaften verleihen, wie z.B. eine erhöhte Resistenz gegenüber Pathogenen (beispielsweise gegenüber phytopathogenen Pilzen, Bakterien, Viren, etc.); eine Resistenz gegenüber Chemikalien [beispielsweise gegenüber Herbiziden (wie z.B. Triazinen, Sulfonylharnstoffen, Imidazolinonen, Triazolpyrimidinen, Bialaphos, Glyphosate, etc,), Insektiziden oder anderen Bioziden]; Resistenz gegenüber nachteiligen (endaphischen oder atmosphärischen) Umwelteinflüssen (beispielsweise gebenüber Hitze, Kälte, Wind, ungünstigen Bodenverhältnissen, Feuchtigkeit, Trockenheit, etc.); oder aber zu einen erhöhten Bildung von Reserve- oder Speicherstoffen in Blättern, Samen, Knollen, Wurzel, Stengeln, etc. führen. Zu den wünschenswerten Substanzen, die von transgenen Pflanzen produziert werden können, zählen beispielsweie Proteine, Stärken, Zukker, Aminosäuren, Alkaloide, Riechstoffe, Farben, Fette, etc..

Eine Resistenz gegenüber Cytotoxinen kann beispielsweise durch Übertragung eines Gens erreicht werden, das in der Lage ist, bei der Expression in der pflanzlichen Zelle, ein Enzym zur Verfügung zu stellen, welches das Cytotoxin detoxifiziert, wie z.B. die Neomycinphosphotransferase Typ 11 oder die Aminoglycosidphosphotransferase Typ IV, die zu einer Detoxifikation von Kanamycin, Hygromycin und anderen Aminoglykosidantibiotika beitragen oder eine Glutathion-S-Transferase, Cytochrom P-450 oder andere katabolisch wirksame Enzyme, von denen bekannt ist, dass sie Triazine, Sulfonylharnstoffe oder andere Herbizide detoxifizieren. Eine Resistenz gegenüber Cytotoxinen kann auch durch ein Gen vermittelt werden, das in einer Pflanze eine bestimmte Form eines 'Zielenzyms' (Angriffspunkt der Cytotoxinwirkung) exprimiert, die resistent ist gegen die Aktivität des Cytotoxins, wie z.B. eine Variante der Acetohydroxysäuresynthase, die gegenüber der inhibitorischen Wirkung von Sulfonylharnstoffen, Imidazolinonen oder anderen Herbiziden, die mit diesem spezifischen Stoffwechselschritt interagieren, insensitiv ist; oder eine Variante der EPSP Synthase, die sich gegenüber der Hemmwirkung von Glyphosate als insensitv erweist. Es kann sich als vorteilhaft erweisen, diese veränderten Zielenzyme in einer Form zu exprimieren, die ihren Transport in das korrekte zelluläre Kompartiment, wie z.B. im obigen Fall in die Chloroplasten, erlaubt.

Im bestimmten Fällen kann es von Vorteil sein, die Genprodukte in die Mitochondrien, die Vakuolen, das endoplasmatische Retikulum oder in andere Zellregionen, möglicherweise sogar in die interzellulären Räume (Apoplasten) zu dirigieren.

Ein Resitenz gegenüber bestimmten Pilzklassen kann beispielsweise durch die Einschleusung eines Gens erreicht werden, das Chitinase in den pflanzlichen Geweben exprimiert. Zahlreiche pflanzenpathogene Pilze enthalten Chitin als integralen Bestandteil ihre Hyphen- und Sporenstukturen, so z.B. die Basidiomyceten (Brand- und Rostpilze), Ascomyceten und Fungi Imperfecti (einschliesslich Altemaria und Bipolaris, Exerophilum turcicum, Colletotricum, Gleocercospora und Cercospora). Chitinase ist in der Lage das Mycelwachstum bestimmter Pathogene in-vitro zu hemmen. Ein pflanzliches Blatt oder eine Wurzel, die Chitinase konstitutiv oder aber als Antwort auf das Eindringen eines Pathogens exprimiert, ist gegen den Angriff einer grossen Zahl verschiedener Pilze geschützt. Je nach Situation kann eine konstitutive Expression vorteilhaft sein im Vergleich zu einer induzierbaren Expression, die bei zahlreichen Pflanzen als normale Reaktion auf einen Pathogenbefall auftritt, da die Chitinase unmittelbar in hoher Konzentration vorliegt, ohne dass erst eine lag-Phase für die Neusynthese abgewartet werden muss.

Eine Resistenz gegenüber Insekten kann beispielsweise durch ein Gen übertragen werden, das ein Polypeptid kodiert, welches toxisch ist für Insekten und/oder deren Larven, wie z.B. das kristalline Protein von Bacillus thuringiensis [Barton et al, 1987; Vaeck et al, 1987]. Eine zweite Klasse von Proteinen, die eine Insektenresistenz vermitteln, sind die Proteaseinhibitoren. Proteaseinhibitoren bilden einen gewöhnlichen Bestandteil von pflanzlichen Speicherstrukturen (Ryan, 1973). Es konnte nachgewiesen werden, dass ein aus Sojabohnen isolierter und gereinigter Bowman-Birk Proteaseinhibitor die Darmprotease von Tenebrio Larven hemmmt (Birk et al, 1963). Das Gen, welches den Trypsininhibitor aus der Kuherbse kodiert, ist bei Hilder et al, (1987) beschrieben.

Ein Gen, das einen Proteaseinhibitor kodiert, kann in einem geeigneten Vektor unter die Kontrolle eines Pflanzenpromotors, insbesondere eines konstitutiven Promotors, wie z.B. des CaMV 35S Promotors [der bei Odell et al, (1985) beschrieben ist], gebracht werden. Das Gen, beispielsweise die kodierende Sequenz des Bowman-Birk Proteaseinhibitors aus der Sojabohne, kann mit Hilfe der bei Hammond et al, (1984) beschriebenen cDNA Klonierungsmethode erhalten werden. Eine weitere Möglichkeit zur Herstellung eines Proteaseinhibitors besteht in dessen synthetischer Herstellung, sofern dieser weniger als 100 Aminosäuren aufweist, wie z.B. der Trypsininhibitor der Limabohne. Die kodierende Sequenz lässt sich durch Zurückübersetzen der Aminosäuresequenz voraussagen. Zusätzlich werden an beiden Enden Restriktionsschnittstellen eingebaut, die für den jeweils gewünschten Vektor geeignet sind. Das synthetische Gen wird durch Synthese überlappender Oligonuldeotidfragmente von 30 bis 60 Basenpaaren hergestellt, indem diese zunächst einer Kinasereaktion unterworfen, dann miteinander verknüpft (Maniatis et al, 1982) und schliesslich in einem passenden Vektor kloniert werden. Mit Hilfe der DNA Sequenzierung kann dann ein Klon, der das Insert in einer korrekten Orientierung aufweist, identifiziert werden. Für die Einschleusung in die Protoplasten wird isolierte Plasmid-DNA verwendet (Abel et al, 1986).

Ebenfalls umfasst von der vorliegenden Erfindung sind Gene, die pharmazeutisch aktive Bestandteile kodieren, z.B. Alkaloide, Steroide, Hormone und andere physiologisch aktive Substanzen sowie Flavine, Vitamine und Farbstoffe. Zu den Genen, die im Rahmen dieser Erfindung in Betracht gezogen werden, gehören daher, ohne jedoch darauf beschränkt zu sein, pflanzenspezifische Gene wie z.B. das Zeingen (Wienand et al, 1981), Säuger-spezifische Gene wie das Insulingen, das Somatostatingen, die Interleucingene, das t-PA Gen (Pennica et al, 1983) etc., oder aber Gene mikrobiellen Ursprungs wie das NPT II Gen sowie synthetische Gene wie das Insulingen (Itakura et al, 1975).

Für die Transformation der Protoplasten kann neben kodierender DNA selbstverständlich auch nicht-kodierende DNA verwendet werden, die in der Regel regulative Funktionen aufweist und z.B. bei der Regualtion des Transkriptionsprozesses involviert sein kann.

Es gibt einzelne Pflanzengene, von denen bekannt ist, dass sie durch verschiedene interne und externe Faktoren wie Pflanzenhormone, Hitzeschock, Chemikalien, Pathogene, Sauerstoffmangel und Licht, induziert werden.

Als ein Beispiel für eine Genregulation durch ein Pflanzenhormon soll die Abszissinsäure (ABS) herangezogen werden, von der bekannt ist, dass sie den während der späten Embryonalphase auftretenden Überschuss an mRNAs in Baumwolle induziert

Ein weiteres Beispiel liefert die Gibberellinsäure (GA3), die in Rizinussamen Malatsynthasetranskripte sowie in den Aleuronschichten von Gerste Isoenzyme der a-Amylase induziert.

Die Regulation von Hitzeschock empfindlichen Proteingenen der Sojabohne wurde im Detail untersucht. Eine mehrstündige Behandlung der Pflanzen bei einer Temperatur von 40° C resultiert in der de novo Synthese von sogenannten Hitzeschock-Proteinen. Zahlreiche dieser Gene wurden inzwischen isoliert und ihre Regulation im einzelnen analysiert. Die Expression dieser Gene wird in erster Linie auf der Ebene der Transkription kontrolliert (Shoffl et al, cited in Willmitzer, 1988). Der Promotor des hps70 Genes wurde mit den Neomycinphosphotransferase II(NPT 11) Gen fusioniert. Es konnte dabei nachgewiesen werden, dass das chimäre Gen durch einen Hizeschock induziert werden kann (Spena et al, 1985).

Eine andere Klasse von in Pflanzen induzierbaren Genen beinhaltet die Licht-regulierten Gene, insbesondere das nukleär kodierte Gen der kleinen Untereinheit der Ribulose-1,5-bisphosphatcarboxylase (RUBISCO). Morelli et al, (1985) und Herrera-Estrella et al, (1984) haben nachgewiesen, dass die 5'-flankierende Sequenz eines RUBISCO-Gens aus der Erbse in der Lage ist, die Lichtinduzierbarkeit auf ein Reportergen zu übertragen, sofern dies in chimärer Form mit dieser Sequenz verknüpft ist. Diese Beobachtung konnte auch auf andere Licht-induzierte Gene ausgedehnt werden, wie z.B. das Chlorophyll a/b - Bindungsprotein.

Die Alkoholdehydrogenase-Gene (adh-Gene) des Mais waren Gegenstand intensiver Untersuchungen. Das adh 1 - s Gen aus Mais wurde isoliert und es wurde nachgewiesen, dass ein Teil der 5'-flankierenden DNA in der Lage ist, die Expression eines chimären Reportergens (z.B. Chloramphenicolacetyltransferase; CAT) zu induzieren, wenn das vorübergehend transformierte Gewebe anaeroben Bedingungen ausgesetzt wurde (Howard et al, 1987)

In einer bevorzugten Ausführungsform der vorliegenden Erfindung werden Protoplasten, die von Pflanzen aus der Unterfamilie Pooideae gewonnen wurden, mit Hilfe einer Kombination aus Elektroporation und Polyethylenglykolbehandlung transformiert. Unmittelbar nach der Reinigung der in Verfahrensschritt B) erhaltenen Protoplasten werden diese einer Elektroporation unterworfen [ vgl. Shillito et al, (1985) sowie EP-0.164,575 (Paszkowski et al)]. Die Protoplasten werden nach dem letzten Spühlgang in einem Elektroporationspuffer resuspendiert. Geeignet im Rahmen dieser Erfindung ist beispielsweise eine Mannitlösung mit einer angemessenen MgCI₂ Konzentration. Zu der Protoplastensuspension wird dann eine wässrige DNA-Lösung zugegeben. In einer spezifischen Ausführungsform dieser Erfindung handelt es sich dabei um das Plasmid pCIB709, das zuvor durch Behandlung mit einer geeigneten Restriktionsendonuklease linearisiert wurde. Die resultierende Mischung wird vorsichtig gerührt. In einer spezifischen Auführungsform dieser Erfindung wird ein halbes Volumen einer 24%igen (w/v) PEG Lösung in 0.5 M Mannit und 30 mM MgCI₂ hinzugegeben. Nach gründlicher Durchmischung werden die Protoplasten in die Kammer eines Dialog@ Elektroporators (DIA-LOG G.m.b.H., Haffstrasse 34, D-4000 Düsseldorf 13, FRG) übertragen und mit 2 bis 10 Pulsen, vorzugsweise aber mit 3 Pulsen von ca. 2'000 V/cm bis 5'000 V/cm Ausgangsspannung und einer exponentiellen Abklingkonstanten von 10 µsec in 30 Sekunden Intervallen beaufschlagt. Die Probe wird dann in eine Petrischale überführt, wobei 1% (w/v) bis 25% (w/v) Agarose als Geliermittel zugesetzt werden. Die Protoplasten werden vor dem Aushärten der Agarose gleichmässig über das Medium verteilt. Aus dieser Kultur von transformierten Protoplasten werden transgene Pflanzen, einschliesslich fertiler transgener Pflanzen aus der Unterfamilie Pooideae gemäss der Beschreibung in den Abschnitten C) bis F) regeneriert.

In einer weiteren bevorzugten Ausführungsform der vorliegenden Erfindung werden die Pooideae Protoplasten gemäss der bei Negrutiu et al, (1987) beschriebenen Methode transformiert. In diesem Fall werden die gereinigten Protoplasten nach dem letzten Spühlgang in einer 0.5 M Mannitlösung suspendiert, die zwischen 15 mM und 45 mM MgCl₂ enthält. Die DNA wird in Form einer wässrigen Lösung zugegeben, gefolgt von einem gleichem Volumen einer 36%igen (w/v) PEG Lösung (Negrutiu et al, 1987). Die resuliterende Lösung wird sorgfältig vermischt und für einen Zeitraum von 5 bis 60 Minuten, vorzugsweise von etwa 30 Minuten, bei einer Temperatur zwischen 10°C und 32°C, vorzugsweise bei Zimmertemperatur (ca. 25°C), inkubiert. Während der Inkubationsphase wird die Lösung gelegentlich bewegt. Nach Beendigung der Inkubation werden die Protoplasten gewaschen und auf einem geeigneten Kulturmedium ausplattiert. Zu den geeigneten Kulturmedien zählt beispielsweise ein KM-8p Medium, ohne jedoch darauf beschränkt zu sein, mit 0.3% (w/v) bis 2.5% (w/v), vorzugsweise aber mit 0.6% (w/v) bis 2% (w/v) Agarose als Verfestigungsmittel. Die Protoplasten werden vor dem Aushärten der Agarose gleichmässig über das Medium verteilt. Aus dieser Kultur von transformierten Protoplasten werden transgene Pflanzen, einschliesslich fertiler transgener Pflanzen aus der Unterfamilie Pooideae gemäss der Beschreibung in der Abschnitten C) bis F) regeneriert.

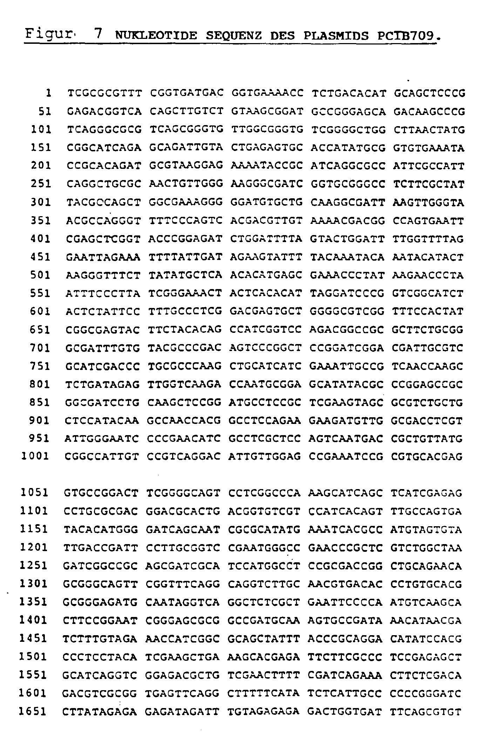

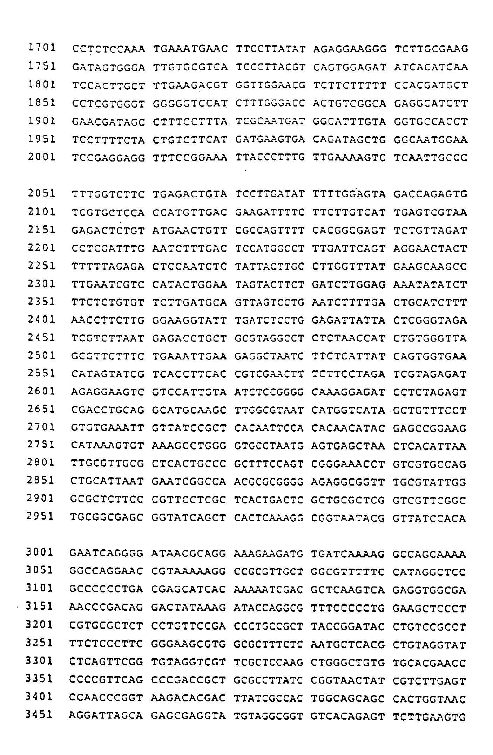

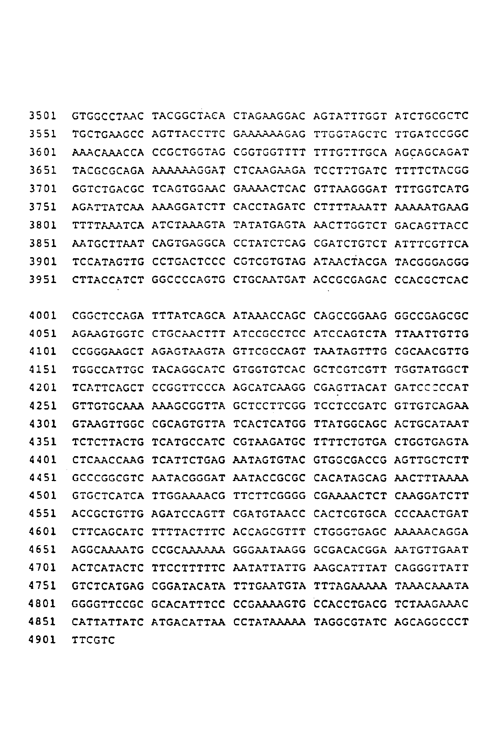

Eine im Rahmen dieser Erfindung bevorzugt exogene DNA ist das in Figur 7 abgebildete Plasmid pCIB709 in linearisierter Form.

### Verfahrenschritt G: Selektionierung transformierter Kolonien

Das mit Agarose verfestigte Medium aus Verfahrensschritt F), das die transformierten Protoplasten enthält, wird im Licht oder aber vorzugsweise im Dunkeln über einen Zeitraum von 5 bis 30 Tagen, vorzugsweise von 8 bis 15 Tagen und ganz besonders bevorzugt von 10 Tagen, bei einer Temperatur zwischen 0°C und 50°C, vorzugsweise zwischen 20°C und 32°C und ganz besonders bevorzugt zwischen 25°C und 28°C, inkubiert. Das Festmedium wird dann beispielsweise in 5 Abschnitte zerschnitten und wie bei Shillito et al, (1983); in der Europäischen Patentanmeldung EP-0,129,688 (Shillito ef al; bei Shillito et al, (1985) oder in der Europäischen Patentanmeldung EP-0,164,575 (Paszkowski et al beschrieben, in einem 'bead type culture system' selektioniert. Anzahl und Grösse der Mediensegmente sind nicht kritisch. In einer spezifischen Ausführungsform dieser Erfindung werden 4 dieser Segmente einzeln in ein geeignetes Medium überführt, wie z.B. ein SH-45 Kulturmedium mit 4 g/Liter Caseinhydrolysat und 20 gg/ml bis 100 gg/ml Hygromycin B. Das 5. Segment wird in das gleiche Medium ohne Hygromycin eingebracht (Kontrolle).

Nach ca. 4 bis 5 Wochen werden die vermutlich transformierten Zellkolonien aus der Agarose ausgeschnitten und in einem geeigneten Kulturmedium, wie beispielsweise einem SH-45 Medium, mit 20 gg/ml bis 100 µg/ml Hygromycin B kultiviert, das auf einer Rundschüttelmaschine bei einer Umdrehungsgeschwindigkeit von 50 Upm bis 80 Upm bewegt wird. Nach weiteren 4 bis 5 Wochen werden sämtliche Kolonien, die eine neue Suspensionskultur liefern, auf frisches Medium mit 20 gg/ml Hygromycin B transferiert. Von den neuen Suspension werden mindestens 2 weitere Folgekulturen in Gegenwart von 20 gg/ml Hygromycin B angelegt. Diese werden unter der gleichen Bedingungen wie zuvor beschrieben kultiviert, bis die Ausbildung von Kallus einsetzt.

Kallus, Suspensionskulturen sowie die Kulturen, die aus den in diesem Verfahrensschritt hergestellten Materialien gewonnen werden können, lassen sich gemäss der in Verfahrensschritt A) beschriebenen Methode in der Kälte konservieren.

### Verfahrensschritt H: Regeneration transformierter Pflanzen aus der Unterfamilie Pooideae aus Kallus

Aus transformiertem Kallus [Verfahrensschritt G)] lassen sich in Übereinstimmung mit dem in den Abschnitten D) bis E) beschriebenen Verfahren transformierte Pflanzen aus der Unterfamilie Pooideae regenerieren.

Das erfindungsgemässe Verfahren gestattet somit eine Regeneration von Protoplasten, die von Gramineen-Pflanzen aus der Unterfamilie Pooideae stammen, zu ganzen Pflanzen, insbesondere aber zu ganzen fertilen Pflanzen. Dies macht es erstmals möglich, exogene DNA stabil in das Genom dieser Pflanzen einzubauen und dadurch ihre Geno- bzw. Phänotypen zu verändern. Darüberhinaus können die Protoplasten intra- oder interspezifisch mit anderen Protoplasten fusioniert werden, wodurch neue Kombinationen nukleärer DNA oder aber neue Kombinationen aus nukleärer und extranukleärer DNA produziert werden. Weiterhin können diese Protoplasten als klonierfähiges Material verwendet werden, an dem Mutations- und Selektionsschritte zur Herstellung eines gewünschten Phänotyps durchgeführt werden können.

Beispiele wünschenswerter Phänotypen umfassen Resistenzen gegenüber toxischen Konzentrationen an natürlichen oder synthetischen Chemikalien, einschliesslich Insektiziden, Herbiziden, Fungiziden, Bakteriziden, Schwermetallen, Salzen, Phatotoxinen, Stoffwechselinhibitoren sowie struktureller oder funktioneller Analoga zellulärer Metabolite, ohne jedoch auf diese beschränkt zu sein. Weitere Beispiele wünschenswerter Phänotypen, auf die selektioniert werden kann, beinhalten Resistenzen gegen widrige Umwelteinflüsse wie kalte oder warme Temperaturen oder biotische Agenzien wie Pathogene.

Die folgenden Ausführungsbeispiele dienen lediglich der weiteren Illustration der vorliegenden Erfindung.

### Ausführungsbeispiele

### Beispiel 1: Herstellung embryogener Suspensionskulturen aus Gewebe von Dactvlis glomerata L. (Knaulgrass)

Als Ausgangsmaterial für den embryogenen Kallus dienen die basalen Abschnitte der jüngsten Blätter von im Gewächshaus gezogenen Knaulgrasspflanzen (Dactylis glomerata L.), wie bei Hanning et al, (1982) beschrieben. Die Blätter werden durch 10 minütiges Eintauchen in eine 1:10 verdünnte Cloroxlösung [eine Lösung von 5,25% (w/v) Natriumhypochlorit; The Clorox Company, Oakland, CA94623, USA] Oberflächen-sterilisiert und anschliessen unter sterilen Bedingungen in kleine Segmente von 1 mm bis 5 mm Länge oder Durchmesser zerschnitten. Diese Segmente werden auf ein steriles SH-30 Medium, das 0.8% (w/v) Agarose als Verfestigungsmittel enthält, ausplattiert. Bei einer Kultivierungstemperatur von ca. 25°C erscheint der Kallus und/oder embryogene Strukturen innerhalb von 2 bis 6 Wochen nach dem Ausplattieren.

Als Ausgangsmaterial für die Herstellung von embryogenen Suspensionskulturen dient embryogener Kallus, von dem eine Menge von ca. 0.5 g (Frischgewicht) in 50 ml eines bei Gray et al, (1985) beschriebenen Flüssigmediums gegeben werden, das 45 µM Dicamba und 4 g/Liter Caseinhydrolysat enthält. Die Suspensionskulturen werden in 125 ml Delong Flaschen, die mit Metallkappen und Parafilm^{®} verschlossen sind, bei einer Temperatur von 27°C und einem Licht-/Dunkelrhythmus von 16 Stunden Licht (40 µE/m²s) und 8 Stunden Dunkelheit auf einer Rundschüttelmaschine bei ca. 130 Upm inkubiert. Nach etwa 4 Wochen lässt man die grossen Zell- Verklumpungen für einen Zeitraum von ca. 30 Sekunden absetzen und entnimmt anschliessend 10 ml Aliquots aus dem Überstand, der kleine Zellkluster enthält und überträgt diese auf 50 ml Frischmedium. Diese Prozedur wird alle 3 bis 4 Wochen wiederholt, wobei jeweils die erfolgversprechendsten Zellkluster verwendet werden, gemessen an der geringeren Klustergrösse und der besseren Qualität, die sich aus der Gegenwart kleiner, cytoplasmareicher Zellen ergibt. Nach 5 bis 8 Übertragungen sind die Suspensionen im wesentlichen frei von nicht embryogenen Zellen und die überwiegende Mehrzahl der embryogenen Zellkluster ist relativ klein (150 wm bis 2'000 µm)

### Beispiel 2: Isolierung und Reinigung von Dactylis glomerata L. Protoplasten

Protoplasten werden aus der embryogenen Suspensionskultur (Beispiel 1) gewonnen, wobei zunächst die Zellen mit Hilfe einer Sterilfiltration über eine Nalgene^{®} 0.2 µm Filtereinheit abgetrennt und anschliessend in einer Menge von 0.5 g (Frischgewicht) zu jeweils 12.5 ml einer in einer Petrischale befindlichen Protoplasten-Enzymmischung zugegeben werden. Die Enzymmischung die aus

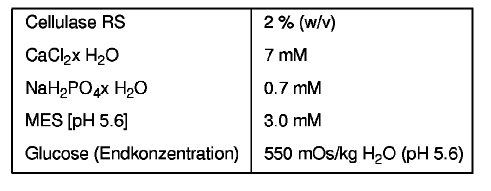

besteht wird anschliessend Filter-sterilisiert. Die Mischung wird auf einer Schüttelmaschine bei einer Geschwindigkeit von ca. 50 Upm bei Dämmerlicht (<5 µE/m²s) für 4 bis 5 Stunden bewegt. Das verdaute Material wird dann durch eine rostfreies Stahlsieb (100 µm Maschenweite) filtriert und auf 12 ml Zentrifugenröhrchen verteilt, die 5 Minuten bei ca. 60 g bis 100 g zentrifugiert werden. Das Protoplasten-haltige Sediment wird dann dreimal mit dem Protoplasten Kulturmedium KM-8p, das mit Hilfe von Glucose auf einen osmotischen Wert von 550 mOs/kg H₂0 eingestellt wird, gewaschen. An dieser Stelle kann ein Flotationsschritt zur weiteren Reinigung der Protoplasten eingebaut werden. In diesem Fall werden die gewaschenen Protoplasten auf die Oberfläche eines mit Saccharose auf einen osmotischen Wert von 700 mOs/kg H₂0 eingestellten KM-8p Mediums (10 ml) plaziert. Nach 10 minütiger Zentrifugation bei 60 g bis 100 g werden die an der Grenzfläche konzentrierten Protoplasten mit Hilfe feiner Pipetten gesammelt. Abschliessend werden die Protoplasten in 1 ml bis 2 ml KM-8p Kulturmedium resuspendiert und durch ein rostfreies Maschengitter (20 µm) filtriert. Die freigesetzten Protoplasten werden gesammelt, gewaschen und für die Kultivierung in KM-8p Medium oder aber in einem osmotisch angepassten Medium, das für eine Transformation gemäss Beispiel 6 geeignet ist, resuspendiert.

### Beispiel 3: Protoplastenkultur von Dactylis alomerata L. und Kalluswachstum

(a) Die gereinigten Protoplasten werden in einer Dichte von etwa 5 x 10⁵ Protoplasten/ml auf KM-8p Kulturmedium ausplattiert, das 1.3% (w/v) SeaPlaque^{®} Agarose (FMC Corp., Marine Colloids Division, Rockland, Maine, USA) and 30% bis 40% (v/v) eines konditionierten Mediums enthält. Das konditionierte Medium wird aus 3 bis 4 Wochen alten embryogenen Suspensionskulturen von Dactylis glomerata L. gewonnen, indem diese über einen sterilen Nalgene^{®} Filter (0.2 pm) filtriert werden, das Medium durch Zugabe von Glucose auf einen osmotischen Wert von 550 mOsm/kg H₂0 eingestellt und anschliessend erneut über eine Filtration sterilisiert wird. Die Platten werden dann im Dunkeln bei einer konstanten Temperatur von 28°C inkubiert. Nach 10 bis 14 Tagen wird die Agarose in keilförmige Stücke zerschnitten und in ein 'bead culture system' (Shillito et al, 1983) eingebracht, unter Verwendung von 20 ml SH-45 Suspensionskulturmedium mit 3% (w/v) Saccharose pro 3 ml der ursprünglichen, Agarose verfestigten Kultur. Die Platten werden auf eine Schüttelmaschine gegeben und bei ca. 50 Upm und einer Beleuchtungsintensität von 8 µE/m²s bewegt. Durch die Freisetzung von Zellen aus der Agarose in das umgebende Flüssigmedium kommt es zur Ausbildung neuer Suspensionskulturen. Die resultierenden, in Suspension kultivierten Zellen, werden auf mit Agar verfestigtem SH-30 Medium ausplattiert und bis zur Ausbildung von Kallus im Dunkeln bei 25°C kultiviert.
(b) Die Protoplasten werden wie zuvor in Beispiel 3(a) beschrieben kultiviert, mit der Ausnahme, dass das Kulturmedium zusätzlich 100 mg/Liter O-Acetylsalicylsäure enthält.
(c) Die Protoplasten werden wie zuvor in Beispiel 3(a) beschrieben kultiviert, mit der Ausnahme, dass das Kulturmedium zusätzlich 30 mg/Liter O-Acetylsalicylsäure enthält.
(d) Die Protoplasten werden wie zuvor in den Beispielen 3(a) bis 3(c) beschrieben kultiviert, mit der Ausnahme, dass das Kulturmedium kein konditioniertes Medium enthält.

### Beispiel 4: Regeneration von Dactylis glomerata L. Pflanzen aus Kallus, der sich von Protoplasten ableitet.

(a) Dactylis glomerata L. Kallus (erhältlich gemäss Beispiel 3), der aus Protoplasten gewonnen wurde, wird auf einem verfestigten SH-30 Medium kultiviert. Alle zwei Wochen werden Folgekulturen angelegt. Alle Embryonen, die sich während dieser Zeit entwickeln, werden entnommen, auf ein Keimungsmedium (SH-0) ausplattiert und im Licht (45 µE/m²s bis 55 µE/m²s) inkubiert. Die Keimung dieser Embryonen erfolgt nach 1 bis 4 Wochen. Die resultierenden Jungpflänzchen werden zur Ausbildung eines Wurzelsystems auf SH-0 Medium überführt und dort im Licht inkubiert. Nach Erreichen des Sechs- bis Zwölf-Blattstadiums werden sie ins Gewächshaus verbracht und dort nach und nach ausgehärtet.
(b) Kallus (erhältlich gemäss Beispiel 3), der aus Protoplasten gewonnen wurde, wird auf einem mit 0.24% (w/v) GelRite^{®} verfestigten SH-0 Medium im Licht (45 µE/m²s bis 55 gE/m²s) kultiviert. Alle zwei Wochen werden Folgekulturen angelegt. Die resultierenden Jungpflänzchen werden werden zur Wurzelbildung auf ein 1:1 Gemisch aus SH-0 und OMS Medium, das mit einer Kombination aus 0.12% (w/v) GelRite^{®} und 0.4% (w/v) Agar verfestigt ist, aufgebracht und im Licht kultiviert. Nach Erreichen des Sechs-bis Zwölf-Blattstadiums werden sie ins Gewächshaus verbracht und dort nach und nach ausgehärtet.
(c) Kleine Jungpflänzchen werden, wie zuvor in den Beispielen 4 (a) und 4(b) beschrieben, gewonnen, auf ein OMS Medium mit 0.8% (w/v) Agar übertragen und zur Ausbildung eines Wurzelsystems in Licht kultiviert. Nach Erreichen des Sechs- bis Zwölf-Blattstadiums werden sie ins Gewächshaus verbracht und dort nach und nach ausgehärtet.
(d) Kleine Jungpflänzchen werden, wie zuvor in Beispiel 4 (a) beschrieben, gewonnen und zur Wurzelbildung auf eine 1:1 Gemisch aus SH-0 und OMS Medium, das mit einer Kombination aus 0.12% (w/v) GelRite^{®} und 0.4% (w/v) Agar verfestigt ist, aufgebracht und im Licht kultiviert. Nach Erreichen des Sechs- bis Zwölf-Blattstadiums werden sie ins Gewächshaus verbracht und dort nach und nach ausgehärtet.

### Beispiel 5: Konstruktion von Plasmid pClB709. einem E. coli Replikon mit einem in Pflanzen exprimierbaren Hygromycinresistenzgen [35S/Hyg^{r}

Die kodierende Sequenz des Strukturgens für Hygromycinresistenz wird aus dem Plasmid pLG90 (Gritz and Davies, 1983) in Form eines ca. 1150 Basen umfassenden BamHI Fragmentes isoliert. Das Plasmid pLG90 kann von Linda Gritz [Applied Biotechnology, 80 Rogers St., Cambridge, Mass. 02141.] bezogen werden. Zur Konstruktion von Plasmid pCIB709 wird das isolierte BamHl Fragment in die BamHl Schnittstelle von pCIB710 (Rothstein et al, 1987) eingespleisst. Das Plasmid pCIB710 enthält die regulatorische Region des CaMV (Cauliflower Mosaic Virus) 35S Transkripts, wobei die Promotor- und Terminatorregion durch eine singuläre BamHl Schnittstelle getrennt sind.

Das resultierende Plasmid pCIB709 wurde bei der 'American Type Culture Collection' (Rockville, Maryland, USA) unter der Hinterlegungsnummer ATCC 40428 hinterlegt.

Vor der Verwendung für eine Transformation, kann das Plasmid pCIB709 durch Behandlung mit dem Restriktionsenzym Pvull linearisiert werden. Dieses Konstrukt enthält dann ein Hygromycinresistenzgen (Aminoglykosidphosphotransferase Typ IV) zusammen mit den 5' und 3' Expressionssignalen des CaMV 35S Transkripts aus Cauliflower Mosaik Virus (CaMV) in einem pCU Plasmid. Die Sequenz von pCIB709 ist in Figur 7 wiedergegeben.

### Beispiel 6: Transformation von Dactylis alomerata L. Protoplasten mit Hilfe der Elektroporation

(a) Unmittelbar im Anschluss an die Reinigung der Protoplasten wird eine Elektroporation gemäss der Beschreibung bei Shillito et al, (1985) durchgeführt, unter Verwendung des in Figur 7 wiedergegebenen Plasmids pCIB709 in linearisierter Form. Die Protoplasten werden nach dem letzten Spühlgang in einer Dichte von 7 x 10⁶ Protoplasten/ml in einem Elektroporationspuffer (0.4 M Mannit, 6 mM MgCl₂) resuspendiert. Die Protoplasten werden dann in Aliquots von 0.7 ml in Plastikzentrifugenröhrchen (10 ml) überführt. Anschliessend wird Plasmid-DNA [62 µl Wasser mit Pvull verdautem Plasmid pCIB709 und Ultraschall-behandelter Kalbsthymus-DNA [Sigma] in einer Endkonzentration von 10 µg/ml (pCIB709) bzw. 50 µg/ml (Kalbsthymus-DNA)] zu den Röhrchen zugegeben. Desweiteren werden 0.38 ml einer Polyethylenglykollösung (PEG-Lösung) [24% (w/v) PEG 6'000 in 0.4 M Mannit, 30 mM MgCl₂, 0.1% (w/v) MES (pH5.6)] zugegeben und die Lösung wird sorgfälltig gemischt. Die Protoplastensuspension wird dann in die Kammer eines Dialog® Elektroporators überführt und mit 10 Pulsen, die eine Ausgangsspannung von 3'250 V/cm und eines exponentille Abklingkonstante von 10 µsec aufweisen und in Intervallen von 30 sec appliziert werden, beaufschlagt. Die Probe wird dann aus der Kammer entnommen und in eine Petrischale mit einem Durchmesser von 10 cm gegeben. Nach Zugabe von 10 ml KM-8p Medium mit 1.2% SeaPlaque⁰ Agarose werden die Protoplasten vor dem Aushärten der Agarose gleichmässig über das ganze Medium verteilt.
(b) Beispiel 6(a) wird wiederholt, mit der Ausnahme, dass die Ausgangsspannung in diesem Fall 3'500 V/cm beträgt.
(c) Beispiel 6(a) wird wiederholt, mit der Ausnahme, dass die Ausgangsspannung in diesem Fall 4'000 V/cm beträgt.
(d) Beispiel 6(a) wird wiederholt, mit der Ausnahme, dass die Ausgangsspannung in diesem Fall 5'000 V/cm beträgt.
(e) Beispiel 6(a) wird wiederholt, mit der Ausnahme, dass die Ausgangsspannung in diesem Fall 3'000 V/cm beträgt.
(f) Beispiel 6(a) wird wiederholt, mit der Ausnahme, dass die Ausgangsspannung in diesem Fall 2'500 V/cm beträgt.
(g) Die Beispiele 6(a) bis 6(f) werden wiederholt, mit der Ausnahme, dass PEG mit einem Molekulargewicht von 4'000 verwendet wird.
(h) Die Beispiele 6(a) bis 6(f) werden wiederholt, mit der Ausnahme, dass PEG mit einem Molekulargewicht von 8'000 verwendet wird.
(i) Die Beispiele 6(a) bis 6(h) werden wiederholt, mit der Ausnahme, dass sich die Endkonzentration des PEG in einem Bereich zwischen 10% und 30% (w/v) bewegt.
(j) Die Beispiele 6(a) bis 6(i) werden wiederholt, mit der Ausnahme, dass zusätzlich eine Hitzeschockbehandlung gemäss der Beschreibung bei Shillito et al, (1985) und Potrykus et al, (1985) durchgeführt wird.

### Beispiel 7: Transformation von Dactylis glomerata L. Protoplasten mit Hilfe einer Polyethilenglykolbehandlung

(a) Der PEG vermittelte direkte Gentransfer wird gemäss der Beschreibung bei Negrutiu et al, (1987) durchgeführt. Bei der verwendeten DNA handelt es sich um das linearisierte Plasmid pCIB709.
   Die Protoplasten werden im Anschluss an den letzten Spühlgang in einer 0.5 M Mannitlösung, die 15 mM MgCI₂ enthält, in einer Konzentration von 2 x 10⁶ pro ml suspendiert. Die Protoplastensuspension wird in Form von 1 ml Aliquots auf Plastikzentrifugenröhrchen (10 ml) verteilt. Vor der Zugabe der PEG-Lösung [40% (w/v)] wird zunächst, wie zuvor in Beispiel 6 im einzelnen beschrieben, die DNA zugesetzt. Die Lösungen werden sorgfälltig vermischt und für einen Zeitraum von 30 Minuten bei Zimmertemperatur unter gelegentlichem Schütteln inkubiert. Danach werden 1.4 ml der Waschlösung zugegeben und die einzelnen Bestandteile des Röhrchens sorgfälltig vermischt. Die Waschlösung besteht aus 87 mM Mannit, 115 mM CaCl₂, 27 mM MgCl₂, 39 mM KCI, 7 mM Tris/HCI und 1.7 g/Liter m-inosit, (pH 9.0). In Abständen von vier Minuten werden vier weitere 1.4 ml Aliquots der Waschlösung zugegeben und der gesamte Ansatz wird jeweils gut vermischt. Die Röhrchen werden dann 10 Minuten mit etwa 60 g zentrifugiert und die Überstände verworfen. Die sedimentierten Protoplasten werden in 1 ml KM-8p Medium aufgenommen und in 10 cm Petrischalen überführt. Nach der Zugabe von 10 ml KM-8p Medium mit 1.2% (w/v) SeaPlaque^{®} Agarose werden die Protoplasten vor dem Aushärten der Agarose gleichmässig über das gesamte Medium verteilt.
(b) Die Transformation wird wie in Beispiel 7(a) beschrieben durch geführt, mit der Ausnahme, dass der pH der Waschlösung auf einen Wert von pH 5.6 eingestellt ist.
(c) Die Transformation wird wie in Beispiel 7(a) beschrieben durchgeführt, mit der Ausnahme, dass der pH der Waschlösung auf einen Wert von pH 7.0 eingestellt ist.
(d) Die Transformation wird wie in den Beispielen 7(a) bis 7(c) beschrieben durchgeführt, mit der Ausnahme, dass das verwendete PEG ein Molekulargewicht von 6'000 aufweist.
(e) Die Transformation wird wie in den Beispielen 7(a) bis 7(c) beschrieben durchgeführt, mit der Ausnahme, dass das verwendete PEG ein Molekulargewicht von 2'000 aufweist.
(f) Die Transformation wird wie in den Beispielen 7(a) bis 7(c) beschrieben durchgeführt, mit der Ausnahme, dass das verwendete PEG ein Molekulargewicht von 8'000 aufweist.
(g) Die Transformation wird wie in den Beispielen 7(a) bis 7(f) beschrieben durchgeführt, mit der Ausnahme, dass zusätzlich eine Hitzeschockbehandlung gemäss der Beschreibung bei Shillito et al, (1985) und Potrykus et al, (1985) durchgeführt wird.
(h) Die Transformation wird wie in den Beispielen 7(a) bis 7(g) beschrieben durchgeführt, mit der Ausnahme, dass die Waschlösung aus 154 mM NaCl, 125 mM CaCl₂ und 5 mM Glucose besteht und mit KOH auf einen pH Wert von pH 6.0 eingestellt ist.
(i) Die Transformation wird wie in den Beispielen 7(a) bis 7(g) beschrieben durchgeführt, mit der Ausnahme, dass die Waschlösung aus 0.2 M CaC1₂ und 0.1% (w/v) MES besteht und mit KOH auf einen pH Wert von pH 6.0 eingestellt ist.
U) Die Transformation wird wie in den Beispielen 7(a) bis 7(g) beschrieben durchgeführt, mit der Ausnahme, dass wie Waschlösung aus 0.2 M CaCl₂ und 7 mM Tris/HCL besteht und mit KOH auf einen pH Wert von pH 9.0 eingestellt ist.

### Beispiel 8: Transformation von Dactylis glomerata L. Protoplasten mit Hilfe der Elektroporation oder einer Polyethylenglykolbehandlung

(a) Die Transformation wird wie in den Beispielen 6 und 7 beschrieben durchgeführt, mit der Ausnahme, dass das Plasmid pCIB709 mit dem Restriktionsemzym Bgl geschnitten wird, bevor es für Transformationszwecke verwendet wird.
(b) Die Transformation wird wie in den Beispielen 6 und 7 beschrieben durchgeführt, mit der Ausnahme, dass das Plasmid pCIB709 mit dem Restriktionsemzym Hindlll geschnitten wird, bevor es für Transformationszwecke verwendet wird.

### Beispiel 9: Transformation von Dactylis alomerata L. Protoplasten mit Hilfe der Elektroporation oder einer Polyethylenglikolbehandluna

Die Transformation wird wie in den Beispielen 6, 7 oder 8 beschrieben durchgeführt, mit der Ausnahme, dass die Protoplasten vor der Verteilung der Aliquots auf die einzelnen Zentrifugenröhrchen für die anschliessende Transformation oder aber nach der Verteilung der Aliquots und vor der Zugabe von PEG, für 5 Minuten bei einer Temperatur von 45°C behandelt werden.

### Beispiel 10: Selektionierung der transformierten Kolonien

(a) Die Kulturplatten (Petrischalen) mit den Protoplasten werden für einen Zeitraum von 10 Tagen bei einer Temperatur von ca. 25°C im Dunkeln inkubiert und dann für die sich anschliessende Kultivierung ('bead culture'; Shillito et al, 1983) in 5 gleichgrosse Stücke zerschnitten. 4 dieser Stücke werden jeweils in 20 ml SH-45 Kulturmedium mit 4 g/Liter Caseinhydrolysat und 20 gg/ml Hygromycin B eingebracht. Das 5. Stück wird ebenfalls in 20 ml des gleichen Mediums eingebracht, dass jedoch kein Hygromycin B enthält und somit als nicht selektive Kontrolle dient. Nach 4 bis 5 Wochen werden die aus Protoplasten gewonnenen, vermutlich transformierten Zellkolonien, die in Anwesenheit von Hygromycin B wachsen, aus der Agarose herausgeschnitten und in eine Petrischale mit einem Durchmesser von 19 mm transferiert, die 2 ml flüssiges SH-45 Medium mit 20 µg/ml Hygromycin B enthält. Die Petrischalen werden auf einer Schüttelmaschine bei einer Umdrehungsgeschwindigkeit von etwa 50 Upm bewegt. Nach weiteren 4 bis 5 Wochen werden all diejenigen Kolonien, die Wachstum zeigen und zu neuen Suspensionen führen, in 125 ml Erlenmeyer Flaschen überführt und, abgesehen von der Hygromycinzugabe (20 µg/ml), in gleicher Weise wie die Starterkultur kultiviert.
   Von den neuen Suspensionen werden alle 1 bis 3 Wochen Folgekulturen angelegt unter Verwendung von SH-45 Medium mit 4 g/Liter Caseinhydrolysat und 20 µg/ml Hygromycin B. Darüberhinaus werden Zellen aus diesen Suspensionen auf SH-30 Festmedien mit 20 µg/ml Hygromycin B ausplattiert und bei 25°C im Dunkeln inkubiert. Von den Kalli, die sich aus diesen ausplattierten Zellen entwickeln, werden alle 2 bis 3 Wochen Folgekulturen auf frischem Medium angelegt. Man geht dann davon aus, dass es sich bei denjenigen Zellen, die in Gegenwart von Hygromycin B wachsen, um Transformanten handelt.
(b) Die Selektionierung erfolgt wie in Beispiel 10(a) beschrieben, wobei jedoch in diesem Fall die aus Protoplasten gewonnenen Zellkolonien, die in einem Hygromycin-haltigen Medium wachsen, auf Agarplatten mit SH-30 Medium, das 20 µg/ml Hygromycin B enthält, übertragen und bei einer Temperatur von 25°C im Dunkeln inkubiert werden.

### Beispiel 11: Regeneration von transformierten Dact,vlis alomerata L. Pflanzen

Die Pflanzen werden in gleicher Weise regeneriert, wie dies in Beispiel 4 für das nicht transformierte Material beschrieben wurde.

### Beispiel 12: Extraktion von DNA aus Kallus und Blattgewebe

DNA wird aus Kallus und Blättern regenerierter Pflanzen unter Verwendung einer modifizierten CETAB Methode (Roger und Bendich, 1985) extrahiert. Dieses Verfahren ist hier am Beispiel von Dactylis glomerata L. Pflanzen beschrieben, kann aber mit gleicher Effizienz auch für Gewebe jeder beliebigen anderen Pflanze aus der Unterfamilie Pooideae verwendet werden. Zur Gewinnung von DNA aus dem oben beschriebenen Material kann aber auch jedes andere der überlicherweise verwendeten DNA Extraktionsverfahren verwendet werden

Auf SH-0 Medium und SH-30 Medium gewachsener Kallus wird in Trockeneis tiefgefroren und dann bei der Temperatur von flüssigem Stickstoff zu einem feinen Pulver zerrieben. Das so erhaltene Pulver wird dann in ein auf die Temperatur von flüssigem Stickstoff vorgekühltes Polypropylen Zentrifugenröhrchen (5 ml) überführt. Dabei ist zu beachten, das das Pulver zu keinem Zeitpunkt während der Durchführung dieser Verfahrensschritte auftaut. Das Pulver wird dann über Nacht gefriergetrocknet und anschliessend auf 2.2 ml Eppendorfröhrchen verteilt, wobei in jedes Röhrchen <0.5 ml Pulver gegeben wird. Zu jedem Röhrchen wird 1 ml eines CETAB Extraktionspuffers hinzugegeben; anschliessend werden die Rörchen bei einer Temperatur von 60°C für etwa 30 bis 45 Minuten inkubiert. Nach Abkühlung auf Zimmertemperatur wird 1 ml eines 24:1 Gemisches aus Chloroform und Isoamylalkohol zugegeben und das Ganze gründlich vermischt. Es folgt eines Zentrifugation für 30 Sekunden bei 3'000 Upm in einer Eppendorf Zentrifuge. Danach wird die wässrige Phase in ein frisches Röhrchen überführt, 1/10 Volumen einer 10%igen (w/v) CETAB Lösung zugesetzt und die Chloroform Extraktion wiederholt. Die wässriger Phase wird erneut in ein frisches Röhrchen gegeben und mit einem gleichen Volumen eines Präzipitationspuffers versetzt. Es kommt dann bei Zimmertemperatur zur Ausfällung von DNA und RNA. Man lässt diese über einen Zeitraum von 30 Minuten bis zu 1 Stunde fortschreiten, bevor man die Röhrchen erneut zentrifugiert. Der Überstand wird verworfen und das Präzipitat in einem TE Puffer (siehe unten), der eine hohe Salzkonzentration aufweist, bei einer Temperatur von 65°C für 30 Minuten resuspendiert.

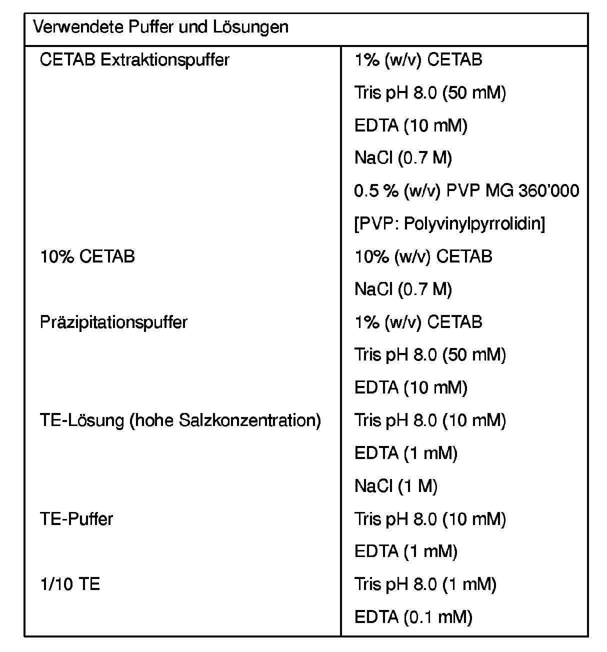

### Beispiel 13: Reinigung der DNA

Die mit Hilfe des in Beispiel 12 beschriebenen Verfahrens oder jedes anderen geeigneten Verfahrens hergestellte DNA kann unter Verwendung eines beliebigen, bekannten Verfahrens gereinigt werden. Beispiele geeigneter Verfahren sind, ohne jedoch darauf beschränkt zu sein : Ethidiumbromid -CsCI Gradientenzentrifugation; Behandlung mit Phenol/Chloroform sowie eine Reinigung über einen Stufengradienten ohne Ethidiumbromid. Diese Verfahren sind bei Maniatis et al, (1982) beschrieben.

### (a) Reinigung durch Phenol/Chloroform Behandlung

Die Nukleinsäuren aus Beispiel 12 werden mit 2 Volumenteilen kaltem Ethanol (-20°C) ausgefällt und die Röhrchen für 2 bis 3 Minuten bei 5'000 g zentrifugiert. Der Überstand wird entfernt und das Präzipitat mit 70%igem und 100%igem Ethanol gewaschen. Danach werden die Nukleinsäuren in einem sterilen Luftstrom teilweise getrocknet. Die DNA wird über Nacht in 200 µl eines 1/10 TE-Puffers gelöst. Die DNA-Lösung wird auf Eppendorf Zentrifugenröhrchen übertragen und es werden 10 µl einer RNAase-Lösung (2 mg/ml), die zuvor erhitzt wurde um etwaig vorhandene DNAase zu desaktivieren, zugesetzt, bevor die Röhrchen bei 37°C für ca. 1 Stunde inkubiert werden. Anschliessend werden 0.25 Volumenteile 5 M NaCI zugegeben und die DNA wird durch Zugabe von 0.4 Volumenteilen einer 30%igen PEG-Lösung (MG 6'000 bis 8'000), die 1.5 M NaCI enthält sowie durch eine einstündige Inkubation bei -20°C ausgefällt. Die Röhrchen werden dann 5 Minuten zentrifugiert, die Überstände verworfen und die Präzipitate mit kaltem absolutem Ethanol gewaschen. Nach kurzem Trocknen in einem sterilen Luftstrom werden die Pellets in 0.3 ml TE-Puffer resuspendiert. Diese Lösung wird mit einem Phenol/Chloroform/lsoamylalkohol(25:24:1)-Gemisch, das mit TE-Puffer äquilibriert ist, extrahiert, 30 Sekunden in einer Eppendorf Zentrifuge zentrifugiert. Die wässrige Phase wird anschliessend auf frische Röhrchen übertragen. Die Lösung wird mit Chloroform/Isoamylalkohol (24:1) extrahiert und 30 Sekunden zentrifugiert und die wässrige Phase anschliessend auf frische Röhrchen übertragen. Die Chloroform-Extraktion wird wiederholt. Danach wird zur Ausfällung der DNA 1/10 Volumen 3 M Natriumacetat zugesetzt, gefolgt von 2 Volumenteilen eisgekühlten Ethanols. Das Präzipitat wird durch Zentrifugation gesammelt, mit 70%igem und 100%igem Ethanol gewaschen, kurz in einem sterilen Luftstrom getrocknet und anschliessend in einer ausreichenden Menge eines TE-Puffers gelöst, sodass man eine Lösung für die 'Southern blot' Analyse erhält, die eine DNA Konzentration zwischen 0.25 µg/µl and 1 µg/µl aufweist.

### (b) Reinigung über einen Stufengradienten ohne Ethidiumbromid

Die Nukleinsäuren werden über einen CsCI Stufengradienten gereinigt, der aus einer 5.7 M CsCI (in TE-Puffer) Unterschicht und einer darübergelagerten Schicht aus 1.0 M CsCI in TE-Puffer besteht. Die Nukleinsäuren werden in die Oberschicht inkorporiert. Die Röhrchen, welche den Stufengradienten enthalten, werden über Nacht in einer Zentrifuge mit exzentrisch rotierendem Rotor (z.B. Beckman SW 50.1; bei 45'000 Upm) zentrifugiert. Die DNA wird aus dem Grenzflächenbereich isoliert, während die RNA vom Boden des Röhrchens gewonnen werden kann. Die DNA wird mit 2 Volumenteilen Wasser verdünnt und mit 2 Volumenteilen eiskalten Ethanols, wie zuvor in Beispiel 13(a) beschrieben, ausgefällt. Die Präzipitate werden dann in TE-Puffer resuspendiert, erneut mit Ethanol präzipitiert und für die 'Southern blot' Analyse verwendet.

### Beispiel 14: Nachweis fremder DNA-Sequenzen im Genom transformierter Dactylis alomerata L. Pflanzen mit Hilfe der 'Southern blot'Analyse.

Die Southern Hybridisierung wird im wesentlichen nach dem bei Maniatis et al, (1982) beschriebenen Verfahren durchgeführt. DNA aus Dactylis glomerata L. Pflanzen (gereinigt nach den in den Beispielen 13(a) und 13(b) beschriebenen Verfahren), wird mit dem Restriktionsenzym BamHl geschnitten. 5 µg davon werden jeweils auf ein 1 %iges Agarosegel aufgetragen und entsprechend ihrer Fragmentgrösse aufgetrennt. Das Gel wird für etwa 20 Minuten in 0.25 M HCI eingebracht, anschliessend mit H₂0 gespült und für weitere 30 Minuten in 0.4 M NaOH gegeben. Die DNA wird dann über Nacht auf die übliche Art und Weise auf GeneScreen Plus® (NEN Res. Products, Kat. Nr. NEF976, Chargen Nr. 330GP62) übertragen (wie in dem Instruktionsheft beschrieben, das mit dem Produkt mitgeliefert wird), wobei 0.4 M NaOH als Transfer-Puffer verwendet wird. Nach dem nächtlichen Transfer wird das Filter entfernt, mit 2 x SSC (0.3 M NaCl, 0.03 M Natriumcitrat) für ca. 5 Minuten gewaschen und anschliessend an der Luft getrocknet. Der Blot wird über einen Zeitraum von 4 Stunden bei einer Temperatur von 65°C mit einem Puffer prähybridisiert, der 10g/Liter Rinderserumalbumin (Fat Free Sigma, Kat. Nr. A-4503), 7% SDS, 1 mM NaEDTA und 0.52 M Natriumphosphat-Puffer (pH 7.0) enthält. Mit Hilfe der 'Random Primer' Methode wird dann unter Verwendung des IBI 'Prime Time' Markierungskits oder einer beliebigen anderen geeigneten Methode, eine radioaktiv marikierte Probe hergestellt, die über eine 'Spin' Säule von überschüssigen Nukleotiden befreit wird. Die DNA-Probe besteht aus einem pCIB709 Fragment, welches die 35S Promotorregion sowie das Strukturgen der Aminoglykosidphosphtransferase Typ IV enthält. Die Hybridisierungsreaktion erfolgt über Nacht bei einer Temperatur von 65°C. Der Blot wird dann 4 mal mit einem SW Wasch-Puffer gespült, wobei die beiden letzten Spühlgänge bei einer Temperatur von 65°C durchgeführt werden. Anschliessend wird der Blot ein weiteres mal gespühlt und zwar über einen Zeitraum von 2 Stunden in 0.2 x SSC mit 1% SDS und 5 mM NaEDTA bei 65°C. Der nasse Blot wird in eine lichtdurchlässige Haushaltsfolie (Saranwrap^{®}) oder in eine beliebige andere geeignete Folie eingeschlagen und mit Hilfe eines Röntgenfilms (Kodak X-Omat AR Film, Eastman Kodak, Rochester, NY 14650, Kat. Nr. 165 1454) entwickelt. Die Auswertung des entwickelten Röntgenfilms lässt eine eindeutige Hybridsierung zwischen der Probe und der DNA, die aus einem mit pCIB709 transformieten Kallus oder aus einer mit pCIB709 transformieten Pflanze gewonnen wurde, erkennen. Die pCIB709 DNA liegt damit eindeutig in die hochmolekulare DNA der Transformaten eingebaut vor.

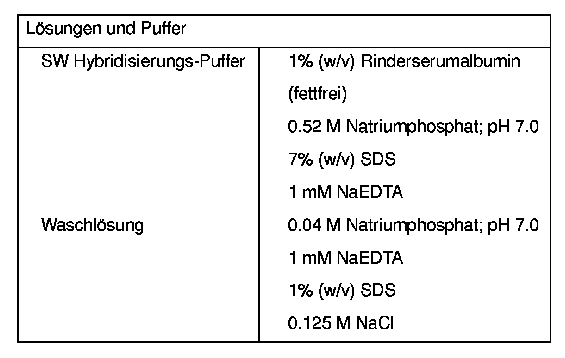

### Literaturverzeichnis

1. Abdullah, R., et al., Bio/Technology, 4 (1986) 1087-1090;
2. Abel, P.P., et al., Science, 233 (1986) 738;
3. Adams, T.L., et al., Plant Cell Reports, 2 (1983) 165-168;
4. Ahloowalia, B.S., Crop Science, 15 (1975) 449-452;
5. Ahloowalia, B.S., Handbook of Plant Cell Culture, Ammirato, et al. (eds) Macmillan, NY, (1984) 91-125;
6. Barton, K.A., et al., Plant Physiol., 85 (1987) 1103-1109;
7. Birk, Y., et al., Biochim. Biophys. Acta, 67 (1963) 326-328;
8. Bright, S.W.J. and Johnes, M.G.K., "Cereal Tissue and Cell Culture: (1985) 204-230, Nijoff, M./Junk, W. Dr., Dordrecht;
9. Brown, W.V, Phytomorphology, 10 (1960) 215-223;
10. Cocking, E.C., and Davey, M.R., Science, 236 (1987) 1259-1262;
11. Fromm, M.E., et al., Nature, 319 (1986) 791-793;
12. Gamborg, O. et al., Exp. Cell Res., 50 (1968) 151-158;
13. George, E.F., et al. (eds), Exgetics Ltd., Edington, Westbury, Wiltshire, England (1987);
14. Gray, D.J., et al., Plant Cell Tissue Organ Cult., 4 (1985) 123-133;
15. Gritz, L., and Davis, J., Gene, 25 (1983) 179-188;
16. Guiseley and Renn, "The Agarose Monograph", Marine Colloids Division, FMC Corp. (1975);
17. Hammond et al., J. Biol. Chem., 259 (1984) 9883-9890;
18. Hanning, G.E., et al., Theor. Appl. Genet., 63 (1982) 155-159;
19. Heide, Physiol. Plantarum, 70 (1987) 523-529;
20. Herrera-Estrella L., et al., Nature, 310 (1984) 115
21. Hilder, V.A., et al., Nature, 330 (1987) 160-163;
22. Howard, et al., Planta, 170 (1987) 535
23. Itakura, K., et al., J. Am. Chem.Soc., 97 (1975) 7327;
24. Kao. K.N., et al., Planta, 126 (1975) 105-110;
25. Kasperbauer, M.J., et al., Crop Science 19 (1979) 457-460;
26. Krans, J.V., et al., Crop Science 22 (1982) 1193-1197;
27. Lipke, H., et al., J. Agr. Food Chem., 2 (1954) 410-414;
28. Lo, P.F., et al., Crop Science, 20 (1980) 363-367;
29. Loerz, H. et al., Mol Gen. Genet., 199 (1985) 178-182;
30. Lu, Ch., et al., Z. Pflanzenphysiol., 104 (1981) 311-318;
31. Luehrs, R., and Loerz, H., Theor. Appl. Genet., 75 (1987) 16-25;
32. Maniatis et al., "Molecular Cloning, a Laboratory Manual", Cold Spring Habor Laboratory (1982);
33. Mettler, l.J., British Patent Application GB-2,140,822;
34. Morelli, et al., Nature, 315 (1985) 200;
35. Murashige, T, et al., Physiol. Plant., (1962) 473-497;
36. Negrutiu, l., et al., Plant Mol. Biology, 8, (1987) 363-373;
37. Odell, J.T. et al., Nature, 313 (1985) 810;
38. Paszkowski, J., et al., The EMBO Journal, 3 (1984) 2717-2722;
39. Paszkowski, J., et al., European Patent Application EP 0,164,575;
40. Pennica, P., et al., Nature 301 (1983) 214;
41. Potrykus, 1., et al., Theor. Appl. Genet., 54 (1979) 209-214;
42. Potrykus, 1., et al., Mon. Gen. Genet., 199 (1985) 183-188;
43. Randolph, L.F., J. Agric. Research, 53 (1936) 881-916;
44. Rhodes, C., et al., Biotechnology, 6 (1988) 56-60;
45. Roger and Bendich, Plant Mol. Biology, 5 (1985) 69-76;
46. Rothstein, S., et al., Gene, 53 (1987) 153-161;
47. Ryan, C., et al., Ann. Rev. Plant Physiol., 24 (1973) 173-196;
48. Shillito, R.D., et al., Plant cell Reports, 2 (1983) 244-247;
49. Shillito, R.D., et al., Bio/Technology, (1985) 1099-1103;
50. Shillito, R.D., et al., European Patent Application EP-0,129,688;
51. Shoffl, F., et al., Plant Cell Environ., cited in Willmitzer L., Trends in Genetics, 4, 13 (1988);
52. Skene, K.G.M., et al., Zeitschr. Pflanzenzüchtung, 90 (1983) 130-135;
53. Spena, et al., EMBO J., 4 (1985) 2736;
54. Stephien, P., et al., Gene, 24 (1983) 281-297;
55. Schenk, R.U. and Hildebrandt, A.C., Can. J. Bot., 50 (1972) 199-204;
56. Vaeck, M., et al., Nature, 328 (1987) 33-37;
57. Vasil, V., et al., Z. Pflanzenphysiol., 111 (1983) 233-239;
58. Wienand, U., et al., Mol. Gen. Genet., 182 (1981) 440-444;
59. Withers, L.A., Plant Tissue Culture and its Agricultural Application;
60. Withers, L.A., and Alderson, P.G. (eds), University Press, Cambridge, England (1986) 261-276;
61. Yamada, Y., et al., Plant Cell Reports, 5 (1986) 85-88.

## Patentansprüche (Patentansprüche für folgende(n) Vertragsstaat(en) : AT, BE, CH, DE, FR, GB, GR, IT, LI, LU, NL, SE)

1. Eine embryogene Zellkultur, die sich von Gramineen-Pflanzen aus der Unterfamilie Pooideae ableitet und von der Protoplasten isoliert werden können, die Zellwände regenerieren, sich teilen und schliesslich einen Kallus ausbilden, der zu ganzen Pflanzen regeneriert werden kann, dadurch erhältlich, dass man
(a) Gewebe aus geeigneten Teilen von Pflanzen aus der Unterfamilie Pooideae isoliert;
(b) dieses Gewebe in einem Medium kultiviert, das in der Lage ist, die Ausbildung eines embryogenen Kallus oder von Embryonen zu induzieren;
(c) von besagtem Kallus und von besagten Embryonen periodisch Folgekulturen auf frischem Medium anlegt, indem man Kleine Zellkluster mit cytoplasmareichen Zellen in frisches Medium überträgt, das in der Lage ist, eine kontinuierliche Proliferation in Gang zu halten; und
(d) nach 0 bis 500 Übertragungen (Transfers) embryogene Zellkluster mit einer Grösse von 150 µm bis 2000 µm isoliert.

2. Eine embryogene Zellkultur gemäss Anspruch 1, dadurch gekennzeichnet, dass es sich bei besagten regenerierten Pflanzen um fertile Pflanzen handelt.

3. Eine embryogene Zellkultur gemäss Anspruch 1, dadurch gekennzeichnet, dass es sich bei besagten Pooideae Pflanzen um Gräser oder um Kleinkömige Getreide handelt.

4. Eine embryogene Zellkultur gemäss Anspruch 3, dadurch gekennzeichnet, dass es sich bei besagten Gräsern um solche ausgewählt aus den Gattungen bestehend aus Poa, Festuca, Lolium, Bromus, Trisetum, Agrostis, Phleum, Alopecurus und Dactylis handelt.

5. Eine embryogene Zellkultur gemäss Anspruch 3, dadurch gekennzeichnet, dass es sich bei besagten kleinkörnigen Getreiden um solche ausgewählt aus den Gattungen Avena, Triticum, Secale und Hordeum handelt.

6. Verfahren zur Herstellung von Protoplasten, die zu ganzen Pflanzen regeneriert werden können, ausgehend von Gramineen-Pflanzen der Unterfamilie Pooideae, dadurch gekennzeichnet, dass man
(a) Gewebe aus geeigneten Teilen von Pflanzen aus der Unterfamilie Pooideae isoliert;
(b) dieses Gewebe in einem Medium kultiviert, das in der Lage ist, die Ausbildung eines embryogenen Kallus oder von Embryonen zu induzieren;
(c) von besagtem embryogenen Kallus und von besagten Embryonen periodisch Folgekulturen auf frischem Medium anlegt, indem man Kleine Zellkluster mit cytoplasmareichen Zellen in frisches Medium überträgt, das in der Lage ist, eine kontinuierliche Proliferation in Gang zu halten;
(d) nach 0 bis 500 Übertragungen (Transfers) embryogene Zellkluster mit einer Grösse von 150 µm bis 2000 µm isoliert; und
(e) mit Hilfe geeigneter Enzyme die Zellwände entfernt und die resultierenden Protoplasten isoliert.

7. Verfahren gemäss Anspruch 6, dadurch gekennzeichnet, dass besagte Pooideae Protoplasten zu ganzen fertilen Pflanzen regeneriert werden können.

8. Verfahren gemäss Anspruch 6, dadurch gekennzeichnet, dass das Gewebe aus Schritt (a) aus den basalen Abschnitten junger, innen liegender Blätter, aus unreifen sexuellen Embryonen, unreifen Blütenständen, reifen Samen oder aus Keimlingsgewebe von Pflanzen aus der Unterfamilie Pooideae isoliert wird.

9. Verfahren gemäss Anspruch 8, dadurch gekennzeichnet, dass das Gewebe aus Schritt (a) aus den jüngsten, am weitesten innen liegenden Blättern isoliert wird.

10. Verfahren gemäss Anspruch 8, dadurch gekennzeichnet, dass die Isolierung der embryogenen Zellkluster nach 0 bis 100 Übertragungen (Transfers) erfolgt.

11. Verfahren gemäss Anspruch 10, dadurch gekennzeichnet, dass die Isolierung der embryogenen Zellkluster nach 3 bis 50 Übertragungen (Transfers) erfolgt.

12. Verfahren zur Herstellung einer Zellkultur, die zu ganzen Pflanzen regeneriert werden kann, ausgehend von Gramineen-Pflanzen der Unterfamilie Pooideae, dadurch gekennzeichnet, dass man
(a) Gewebe aus geeigneten Teilen von Pflanzen aus der Unterfamilie Pooideae isoliert;
(b) dieses Gewebe in einem Medium kultiviert, das in der Lage ist, die Ausbildung eines embryogenen Kallus oder von Embryonen zu induzieren;
(c) von besagtem embryogenen Kallus und von besagten Embryonen periodisch Folgekulturen auf frischem Medium anlegt, indem man Kleine Zellkluster mit cytoplasmareichen Zellen in frisches Medium überträgt, das in der Lage ist, eine kontinuierliche Proliferation in Gang zu halten;
(d) nach 0 bis 500 Übertragungen (Transfers) embryogene Zellkluster mit einer Grösse von 150 µm bis 2000 µm isoliert;
(e) gegebenenfalls mit Hilfe geeigneter Enzyme die Zellwände entfernt und die resultierenden Protoplasten isoliert;
(f) Protoplasten gemäss Verfahrensschritt (e) oder pflanzliche Zellen gemäss Verfahrensschritt (d) bis zur Ausbildung von Zellkolonien in einem geeigneten Kulturmedium kultiviert;
(g) besagte Zellkolonien oder Teile davon auf einem geeigneten Medium, das die Bildung von Zellkulturen fördert, kultiviert; und
(h) die resultierenden Zellkulturen isoliert.

13. Verfahren zur Herstellung einer Zellkultur gemäss Anspruch 12, die zu ganzen Pflanzen regeneriert werden können, ausgehend von Gramineen-Pflanzen der Unterfamilie Pooideae , dadurch gekennzeichnet, dass man
(a) die Protoplasten oder pflanzliche Zellen in einem geeigneten Kulturmedium für einen Zeitraum, der ausreicht um die Bildung von Zellkulturen zu fördern, bis zur Ausbildung von Zellkolonien kultiviert; und
(b) die resultierenden Zellkulturen isoliert.

14. Verfahren zur Herstellung einer Zellkultur gemäss einem der Ansprüche 12 oder 13, dadurch gekennzeichnet, dass besagte Zellkultur zu ganzen fertilen Pflanzen regeneriert werden kann.

15. Verfahren gemäss einem der Ansprüche 12 bis 14, dadurch gekennzeichnet, dass es sich bei besagter Zellkultur um eine Suspensionskultur handelt.

16. Verfahren gemäss einem der Ansprüche 12 bis 14, dadurch gekennzeichnet, dass es sich bei besagter Zellkultur um eine Kalluskultur handelt.

17. Verfahren gemäss einem der Ansprüche 12 oder 13, dadurch gekennzeichnet, dass man Verfahrensschritt (a) in Gegenwart von Agarose als Verfestigungsmittel ausführt.

18. Verfahren gemäss Anspruch 17, dadurch gekennzeichnet, dass man das mit Agarose verfestigte Medium aus Verfahrensschritt (a) verflüssigt oder aber segmentiert und das verflüssigte oder segmentierte Agarosemedium in ein Flüssigmedium überträgt und dort bis zur Ausbildung von Zellkolonien weiterkultiviert.

19. Verfahren gemäss einem der Ansprüche 12 oder 13, dadurch gekennzeichnet, dass es sich bei besagten Teilen einer Zellklonie in Verfahrensschritt (b) um Zellen handelt, die aus den Zellkolonien freigesetzt werden.

20. Verfahren gemäss einem der Ansprüche 12 oder 13, dadurch gekennzeichnet, dass besagte Protoplasten oder Zellen exogene DNA stabil in ihr Genom eingebaut enthalten, die nicht auf natürlichem Weg in das Genom integriert werden kann.

21. Verfahren gemäss Anspruch 20, dadurch gekennzeichnet, dass die integrierte exogene DNA in pflanzlichen Zellen exprimierbar ist.

22. Verfahren zur Regeneration von Gramineen Pflanzen aus der Unterfamilie Pooideae, dadurch gekennzeichnet, dass man
(a) Zellkulturen gemäss Anspruch 12 bis zur Ausbildung von Embryonen kultiviert;
(b) die Embryonen auf einem Medium kultiviert, das geeignet ist, die Reifung und Keimung besagter Embryonen zu induzieren; und
(c) die resultierenden Jungpflänzchen weiterkultiviert, bis sie ein Entwicklungsstadium erreicht haben, das eine Übertragung in Erde erlaubt.

23. Verfahren zur Regeneration von fertilen Gramineen Pflanzen aus der Untertamilie Pooideae, dadurch gekennzeichnet, dass man
(a) Zellkulturen gemäss Anspruch 12 bis zur Ausbildung von Embryonen kultiviert;
(b) die Embryonen auf einem Medium kultiviert, das geeignet ist, die Reifung und Keimung besagter Embryonen zu induzieren;
(c) die resultierenden Jungpflänzchen weiterkultiviert, bis sie ein Entwicklungsstadium erreicht haben, das eine Übertragung in Erde erlaubt; und
(d) im Anschluss an eine kontrollierte oder offene Bestäubung Samen gewinnt.

24. Verfahren gemäss einem der Ansprüche 22 oder 23, dadurch gekennzeichnet, dass besagte Pflanzenzellen, die den Kallus aufbauen, exogene DNA stabil in ihr Genom eingebaut enthalten, die nicht auf natürlichem Weg in das Genom integriert werden kann.

25. Verfahren gemäss Anspruch 24, dadurch gekennzeichnet, dass die integrierte exogene DNA in pflanzlichen Zellen exprimierbar ist.

26. Verfahren zur Herstellung transgener Gramineen Pflanzen aus der Unterfamilie Pooideae, dadurch gekennzeichnet, dass man
(a) Protoplasten gemäss Anspruch 6 mit Hilfe bekannter Transformationsverfahren mit exogener DNA transformiert, die nicht auf natürlichem Weg in das Genom integriert werden kann,
(b) die transformierten Protoplasten gemäss einem der Ansprüche 12 oder 13 kultiviert und
(c) ganze Pflanzen gemäss einem der Ansprüche 22 oder 23 regeneriert

27. Verfahren gemäss Anspruch 26, dadurch gekennzeichnet, dass es sich bei besagter exogener DNA um ein chimäres Gen oder um mehrere chimäre Gene handelt, die den transformierten Protoplasten sowie den sich daraus entwickelnden Geweben und insbesondere den Pflanzen vorteilhafte Eigenschaften verleihen.

28. Verfahren gemäss Anspruch 26, dadurch gekennzeichnet, dass es sich bei besagter exogener DNA um eine nicht-kodierende DNA mit regulatorischer Funktion handelt.

29. Verfahren gemäss Anspruch 26, dadurch gekennzeichnet, dass man in Verfahrensschritt (a) ein direktes Gentransfer-Verfahren für die Protoplastentransformation verwendet.

30. Gramineen-Pflanzen aus der Unterfamilie Pooideae sowie deren Vermehrungsgut, dadurch gekennzeichnet, dass es sich bei besagter Pflanze um eine fertile Pflanze handelt,welche ausgehend von einer embryogenen Zellkultur gemäss einem der Ansprüche 1 bis 5 durch Regeneration erhältlich ist und deren Vermehrungsgut stabil ins pflanzliche Genom integrierte exogene DNA enthält, die in Pflanzen oder pflanzlichen Zellen exprimierbar ist und nicht auf natürlichem Weg in das Genom integriert werden kann.

31. Vermehrungsgut gemäss Anspruch 30, dadurch gekennzeichnet, dass es sich bei besagtem Vermehrungsgut um pflanzliches Material handelt, das sexuell oder asexuell sowie in-vitro oder in-vivo vermehrt werden kann.

32. Vermehrungsgut gemäss Anspruch 31, dadurch gekennzeichnet, dass es sich bei besagtem Vermehrungsgut um Protoplasten, Zellen, Kalli, Gewebe, Organe, Zygoten, Embryonen oder Samen handelt, die von transgenen Pooideae Pflanzen gemäss Anspruch 30 stammen.

33. Nachkommen von Pflanzen gemäss Anspruch 30, deren Genom besagte exogene DNA enthält.

## Patentansprüche (Patentansprüche für folgende(n) Vertragsstaat(en) : ES)

1. Verfahren zur Herstellung von Protoplasten, die zu ganzen Pflanzen regeneriert werden können, ausgehend von Gramineen-Pflanzen der Unterfamilie Pooideae, dadurch gekennzeichnet, dass man
(a) Gewebe aus geeigneten Teilen von Pflanzen aus der Unterfamilie Pooideae isoliert;
(b) dieses Gewebe in einem Medium kultiviert, das in der Lage ist, die Ausbildung eines embryogenen Kallus oder von Embryonen zu induzieren;
(c) von besagtem embryogenen Kallus und von besagten Embryonen periodisch Folgekulturen auf frischem Medium anlegt,indem man Kleine Zellkluster mit cytoplasmareichen Zellen in frisches Medium überträgt, das in der Lage ist, eine kontinuierliche Proliferation in Gang zu halten;
(d) nach 0 bis 500 Übertragungen (Transfers) embryogene Zellkluster mit einer Grösse von 150 µm bis 2000 /-Lm isoliert; und
(e) mit Hilfe geeigneter Enzyme die Zellwände entfernt und die resultierenden Protoplasten isoliert.

2. Verfahren gemäss Anspruch 1, dadurch gekennzeichnet, dass besagte Pooideae Protoplasten zu ganzen fertilen Pflanzen regeneriert werden können.

3. Verfahren gemäss Anspruch 1, dadurch gekennzeichnet, dass das Gewebe aus Schritt (a) aus den basalen Abschnitten junger, innen liegender Blätter, aus unreifen sexuellen Embryonen, unreifen Blütenständen, reifen Samen oder aus Keimlingsgewebe von Pflanzen aus der Unterfamilie Pooideae isoliert wird.

4. Verfahren gemäss Anspruch 3, dadurch gekennzeichnet, dass das Gewebe aus Schritt (a) aus den jüngsten, am weitesten innen liegenden Blättern isoliert wird.

5. Verfahren gemäss Anspruch 3, dadurch gekennzeichnet, dass die Isolierung der embryogenen Zellkluster nach 0 bis 100 Übertragungen (Transfers) erfolgt.

6. Verfahren gemäss Anspruch 5, dadurch gekennzeichnet, dass die Isolierung der embryogenen Zellkluster nach 3 bis 50 Übertragungen (Transfers) erfolgt.

7. Verfahren zur Herstellung einer Zellkultur, die zu ganzen Pflanzen regeneriert werden kann, ausgehend von Gramineen-Pflanzen der Unterfamilie Pooideae, dadurch gekennzeichnet, dass man
(a) Gewebe aus geeigneten Teilen von Pflanzen aus der Unterfamilie Pooideae isoliert;
(b) dieses Gewebe in einem Medium kultiviert, das in der Lage ist, die Ausbildung eines embryogenen Kallus oder von Embryonen zu induzieren;
(c) von besagtem embryogenen Kallus und von besagten Embryonen periodisch Folgekulturen auf frischem Medium anlegt, indem man kleine Zellkluster mit cytoplasmareichen Zellen in frisches Medium überträgt, das in der Lage ist, eine kontinuierliche Proliferation in Gang zu halten;
(d) nach 0 bis 500 Übertragungen (Transfers) embryogene Zellkluster mit einer Grösse von 150 µm bis 2000 µm isoliert;
(e) gegebenenfalls mit Hilfe geeigneter Enzyme die Zellwände entfernt und die resultierenden Protoplasten isoliert;
(f) Protoplasten gemäss Verfahrensschritt (e) oder pflanzliche Zellen gemäss Verfahrensschritt (d) bis zur Ausbildung von Zellkolonien in einem geeigneten Kulturmedium kultiviert;
(g) besagte Zellkolonien oder Teile davon auf einem geeigneten Medium, das die Bildung von Zellkulturen fördert, kultiviert; und
(h) die resultierenden Zellkulturen isoliert.

8. Verfahren zur Herstellung einer Zellkultur gemäss Anspruch 7, die zu ganzen Pflanzen regeneriert werden können, ausgehend von Gramineen-Pflanzen der Unterfamilie Pooideae , dadurch gekennzeichnet, dass man
(a) die Protoplasten oder pflanzliche Zellen in einem geeigneten Kulturmedium für einen Zeitraum, der ausreicht um die Bildung von Zellkulturen zu fördern, bis zur Ausbildung von Zellkolonien kultiviert; und
(b) die resultierenden Zellkulturen isoliert.

9. Verfahren zur Herstellung einer Zellkultur gemäss einem der Ansprüche 7 oder 8, dadurch gekennzeichnet, dass besagte Zellkultur zu ganzen fertilen Pflanzen regeneriert werden kann.

10. Verfahren gemäss einem der Ansprüche 7 bis 9, dadurch gekennzeichnet, dass es sich bei besagter Zellkultur um eine Suspensionskultur handelt.

11. Verfahren gemäss einem der Ansprüche 7 bis 9, dadurch gekennzeichnet, dass es sich bei besagter Zellkultur um eine Kalluskultur handelt.

12. Verfahren gemäss einem der Ansprüche 7 oder 8, dadurch gekennzeichnet, dass man Verfahrensschritt (a) in Gegenwart von Agarose als Verfestigungsmittel ausführt.

13. Verfahren gemäss Anspruch 12, dadurch gekennzeichnet, dass man das mit Agarose verfestigte Medium aus Verfahrensschritt (a) verflüssigt oder aber segmentiert und das verflüssigte oder segmentierte Agarosemedium in ein Flüssigmedium überträgt und dort bis zur Ausbildung von Zellkolonien weiterkultiviert.

14. Verfahren gemäss einem der Ansprüche 7 oder 8, dadurch gekennzeichnet, dass es sich bei besagten Teilen einer Zellklonie in Verfahrensschritt (b) um Zellen handelt, die aus den Zellkolonien freigesetzt werden.

15. Verfahren gemäss einem der Ansprüche 7 oder 8, dadurch gekennzeichnet, dass besagte Protoplasten oder Zellen exogene DNA stabil in ihr Genom eingebaut enthalten, die nicht auf natürlichem Weg in das Genom integriert werden kann.

16. Verfahren gemäss Anspruch 15, dadurch gekennzeichnet, dass die integrierte exogene DNA in pflanzlichen Zellen exprimierbar ist.

17. Verfahren zur Regeneration von Gramineen Pflanzen aus der Unterfamilie Pooideae, dadurch gekennzeichnet, dass man
(a) Zell kulturen gemäss Anspruch 7 bis zur Ausbildung von Embryonen kultiviert;
(b) die Embryonen auf einem Medium kultiviert, das geeignet ist, die Reifung und Keimung besagter Embryonen zu induzieren; und
(c) die resultierenden Jungpflänzchen weiterkultiviert, bis sie ein Entwicklungsstadium erreicht haben, das eine Übertragung in Erde erlaubt.

18. Verfahren zur Regeneration von fertilen Gramineen Pflanzen aus der Unterfamilie Pooideae, dadurch gekennzeichnet, dass man
(a) Zellkulturen gemäss Anspruch 7 bis zur Ausbildung von Embryonen kultiviert;
(b) die Embryonen auf einem Medium kultiviert, das geeignet ist, die Reifung und Keimung besagter Embryonen zu induzieren;
(c) die resultierenden Jungpflänzchen weiterkultiviert, bis sie ein Entwicklungsstadium erreicht haben, das eine Übertragung in Erde erlaubt; und
(d) im Anschluss an eine kontrollierte oder offene Bestäubung Samen gewinnt.

19. Verfahren gemäss einem der Ansprüche 17 oder 18, dadurch gekennzeichnet, dass besagte Pflanzenzellen, die den Kallus aufbauen, exogene DNA stabil in ihr Genom eingebaut enthalten, die nicht auf natürlichem Weg in das Genom integriert werden kann.

20. Verfahren gemäss Anspruch 19, dadurch gekennzeichnet, dass die integrierte exogene DNA in pflanzlichen Zellen exprimierbar ist.

21. Verfahren zur Herstellung transgener Gramineen Pflanzen aus der Unterfamilie Pooideae, dadurch gekennzeichnet, dass man
(a) Protoplasten gemäss Anspruch 1 mit Hilfe bekannter Transformationsverfahren mit exogener DNA transformiert, die nicht auf natürlichem Weg in das Genom integriert werden kann,
(b) die transformierten Protoplasten gemäss einem der Ansprüche 7 oder 8 kultiviert und
(c) ganze Pflanzen gemäss einem der Ansprüche 17 oder 18 regeneriert.

22. Verfahren gemäss Anspruch 21, dadurch gekennzeichnet, dass es sich bei besagter exogener DNA um ein chimäres Ges oder um mehrere chimäre Gene handelt, die den transformierten Protoplasten sowie den sich daraus entwickelnden Geweben und insbesondere den Pflanzen vorteilhafte Eigenschaften verleihen.

23. Verfahren gemäss Anspruch 21, dadurch gekennzeichnet, dass es sich bei besagter exogener DNA um eine nicht-kodierende DNA mit regulatorischer Funktion handelt.

24. Verfahren gemäss Anspruch 21, dadurch gekennzeichnet, dass man in Verfahrensschritt (a) ein direktes Gentransfer-Verfahren für die Protoplastentransformation verwendet.

## Claims (Claims for the following Contracting State(s) : AT, BE, CH, DE, FR, GB, GR, IT, LI, LU, NL, SE)

1. An embryogenic cell culture which is derived from graminaceous plants of the subfamily Pooideae and from which protoplasts can be isolated which regenerate cell walls, divide and finally form a callus capable of being regenerated into complete plants, which is obtainable by:
(a) isolating tissue from suitable parts of plants of the subfamily Pooideae;
(b) culturing this tissue in a medium capable of inducing the formation of an embryogenic callus or of embryos;
(c) periodically subculturing said callus and said embryos on fresh medium by transferring small cell clusters containing cytoplasm-rich cells to fresh medium capable of sustaining continuous proliferation; and
(d) after 0 to 500 transfers isolating embryogenic cell clusters with a size from 150 /-Lm to 2000 wm.

2. An embryogenic cell culture according to claim 1 wherein said regenerated plants are fertile plants.

3. An embryogenic cell culture according to claim 1 wherein the said Pooideae plants are grasses or small-grain cereals.

4. An embryogenic cell culture according to claim 3 wherein said grasses are selected from the genera consisting of Poa, Festuca, Lolium, Bromus, Trisetum, Agrostis, Phleum, Alopecurus and Dactylis.

5. An embryogenic cell culture according to claim 3 wherein said small-grain cereals are selected from the genera Avena, Triticum, Secale and Hordeum.

6. A method of producing protoplasts capable of being regenerated into complete plants, starting from graminaceous plants of the subfamily Pooideae which comprises:
(a) isolating tissue from suitable parts of plants of the subfamily Pooideae;
(b) culturing this tissue in a medium capable of inducing the formation of an embryogenic callus or of embryos;
(c) periodically subculturing said embryogenic callus and said embryos on fresh medium by transferring small cell clusters containing cytoplasm-rich cells to fresh medium capable of sustaining continuous proliferation;
(d) after 0 to 500 transfers isolating embryogenic cell clusters with a size from 150 pm to 2000 pm; and
(e) removing the cell walls with the aid of suitable enzymes and isolating the resultant protoplasts.

7. A method according to claim 6 wherein said Pooideae protoplasts are capable of being regenerated into complete fertile plants.

8. A method according to claim 6 wherein the tissue of step (a) is isolated from the basal parts of young, inner leaves, immature sexual embryos, immature inflorescences, mature seeds or seedling tissue of plants of the Pooideae subfamily.

9. A method according to claim 8 wherein the tissue of step (a) is isolated from the youngest, innermost leaves.

10. A method according to claim 8 wherein the isolation of embryogenic cell clusters is carried out after 0 to 100 transfers.

11. A method according to claim 10 wherein the isolation of embryogenic cell clusters is carried out after 3 to 50 transfers.

12. A method of producing a cell culture capable of being regenerated into complete plants, starting from graminaceous plants of the subfamily Pooideae, which comprises:
(a) isolating tissue from suitable parts of plants of the subfamily Pooideae;
(b) culturing this tissue in a medium capable of inducing the formation of an embryogenic callus or of embryos;
(c) periodically subculturing said embryogenic callus and said embryos on fresh medium by transferring small cell clusters containing cytoplasm-rich cells to fresh medium capable of sustaining continuous proliferation;
(d) after 0 to 500 transfers isolating embryogenic cell clusters with a size from 150 wm to 2000 wm;
(e) removing the cell walls with or without the aid of suitable enzymes and isolating the resultant protoplasts;
(f) culturing in a suitable culture medium step (e) protoplasts or step
(d) plant cells until cell colonies develop;
(g) culturing said cell colonies or parts thereof on a suitable medium which promotes cell culture formation; and
(h) isolating the resultant cell cultures.

13. A method of producing a cell culture according to claim 12 which is capable of being regenerated into complete plants, starting from graminaceous plants of the subfamily Pooideae, which comprises:
(a) culturing in a suitable culture medium the protoplasts or plant cells for a time sufficient to promote cell culture formation until cell colonies develop; and
(b) isolating the resultant cell cultures.

14. A method of producing a cell culture according to one of claims 12 or 13, wherein said cell culture is capable of being regenerated into complete fertile plants.

15. A method according to one of claims 12 to 14 wherein said cell culture is a suspension culture.

16. A method according to one of claims 12 to 14 wherein said cell culture is a callus culture.

17. A method according to one of claims 12 or 13 wherein step (a) is carried out in the presence of agarose as solidifying agent.

18. A method according to claim 17 which comprises liquefying the agarose-solidified medium of step (a) or segmenting it, transferring the liquefied or segmented agarose medium to a liquid medium, and continuing culturing until cell colonies develop.

19. A method according to one of claims 12 or 13 wherein said parts of a cell colony in step (b) are cells released from the cell colonies.

20. A method according to one of claims 12 or 13 wherein said protoplasts or cells contain exogenous DNA integrated stably into their genome, which DNA cannot be integrated into the genome by natural means.

21. A method according to claim 20 wherein the integrated exogenous DNA is expressible in plant cells.

22. A method of regenerating graminaceous plants of the subfamily Pooideae, which comprises:
(a) culturing cell cultures according to claim 12 until embryos develop;
(b) culturing the embryos on a medium suitable to induce them to mature and germinate; and
(c) further culturing the resultant plantlets until sufficiently developed to be transferred to soil.

23. A method of regenerating fertile graminaceous plants of the subfamily Pooideae, which comprises:
(a) culturing cell cultures according to claim 12 until embryos develop;
(b) culturing the embryos on a medium suitable to induce them to mature and germinate;
(c) further culturing the resultant plantlets until sufficiently developed to be transferred to soil; and
(d) obtaining seed following controlled or open pollination.

24. A method according to one of claims 22 or 23 wherein said plant cells forming the callus contain exogenous DNA integrated stably into their genome, which DNA cannot be integrated into the genome by natural means.

25. A method according to claim 24 wherein the integrated exogenous DNA is expressible in plant cells.

26. A method for producing transgenic graminaceous plants of the subfamily Pooideae, which comprises:
(a) transforming protoplasts according to claim 6 with exogenous DNA using known transformation methods, which DNA cannot be integrated into the genome by natural means
(b) culturing the transformed protoplasts according to one of claims 12 or 13, and
(c) regenerating complete plants according to one of claims 22 or 23.

27. A method according to claim 26, wherein said exogenous DNA comprises one or more chimeric genes which provide the transformed protoplasts and the protoplast-derived tissues and, in particular, the plants with advantageous properties.

28. A method according to claim 26, wherein said exogenous DNA comprises a non-coding DNA with a regulatory function.

29. A method according to claim 26, wherein a direct gene transfer method is used in step (a) for the transformation of protoplasts.

30. Graminaceous plants of the subfamily Pooideae and their propagules, wherein said plant is a fertile plant obtainable by regeneration starting from an embryogenic cell culture according to one of claims 1 to 5 and whose propagules comprise exogenous DNA incorporated stably into the plant genome, which DNA is expressible in plants or plant cells and cannot be integrated into the genome by natural means.

31. Propagules according to claim 30, wherein said propagules comprise plant material capable of being propagated sexually or asexually, and in vitro or in vivo.

32. Propagules according to claim 31 wherein said propagules comprise protoplasts, cells, calli, tissue, organs, zygotes, embryos or seeds which originate from transgenic Pooideae plants according to claim 30.

33. Progeny of plants according to claim 30 whose genome comprises said exogenous DNA.

## Claims (Claims for the following Contracting State(s) : ES)

1. A method of producing protoplasts capable of being regenerated into complete plants, starting from graminaceous plants of the subfamily Pooideae which comprises:
(a) isolating tissue from suitable parts of plants of the subfamily Pooideae;
(b) culturing this tissue in a medium capable of inducing the formation of an embryogenic callus or of embryos;
(c) periodically subculturing said embryogenic callus and said embryos on fresh medium by transferring small cell clusters containing cytoplasm-rich cells to fresh medium capable of sustaining continuous proliferation;
(d) after 0 to 500 transfers isolating embryogenic cell clusters with a size from 150 pm to 2000 pm; and
(e) removing the cell walls with the aid of suitable enzymes and isolating the resultant protoplasts.

2. A method according to claim 1 wherein said Pooideae protoplasts are capable of being regenerated into complete fertile plants.

3. A method according to claim 1 wherein the tissue of step (a) is isolated from the basal parts of young, inner leaves, immature sexual embryos, immature inflorescences, mature seeds or seedling tissue of plants of the Pooideae subfamily.

4. A method according to claim 3 wherein the tissue of step (a) is isolated from the youngest, innermost leaves.

5. A method according to claim 3 wherein the isolation of embryogenic cell clusters is carried out after 0 to 100 transfers.

6. A method according to claim 5 wherein the isolation of embryogenic cell clusters is carried out after 3 to 50 transfers.

7. A method of producing a cell culture capable of being regenerated into complete plants, starting from graminaceous plants of the subfamily Pooideae, which comprises:
(a) isolating tissue from suitable parts of plants of the subfamily Pooideae;
(b) culturing this tissue in a medium capable of inducing the formation of an embryogenic callus or of embryos;
(c) periodically subculturing said embryogenic callus and said embryos on fresh medium by transferring small cell clusters containing cytoplasm-rich cells to fresh medium capable of sustaining continuous proliferation;
(d) after 0 to 500 transfers isolating embryogenic cell clusters with a size from 150 wm to 2000 wm;
(e) removing the cell walls with or without the aid of suitable enzymes and isolating the resultant protoplasts;
(f) culturing in a suitable culture medium step (e) protoplasts or step
(d) plant cells until cell colonies develop;
(g) culturing said cell colonies or parts thereof on a suitable medium which promotes cell culture formation; and
(h) isolating the resultant cell cultures.

8. A method of producing a cell culture according to claim 7 which is capable of being regenerated into complete plants, starting from graminaceous plants of the subfamily Pooideae, which comprises:
(a) culturing in a suitable culture medium the protoplasts or plant cells for a time sufficient to promote cell culture formation until cell colonies develop; and
(b) isolating the resultant cell cultures.

9. A method of producing a cell culture according to one of claims 7 or 8, wherein said cell culture is capable of being regenerated into complete fertile plants.

10. A method according to one of claims 7 to 9 wherein said cell culture is a suspension culture.

11. A method according to one of claims 7 to 9 wherein said cell culture is a callus culture.

12. A method according to one of claims 7 or 8 wherein step (a) is carried out in the presence of agarose as solidifying agent.

13. A method according to claim 12 which comprises liquefying the agarose-solidified medium of step (a) or segmenting it, transferring the liquefied or segmented agarose medium to a liquid medium, and continuing culturing until cell colonies develop.

14. A method according to one of claims 7 or 8 wherein said parts of a cell colony in step (b) are cells released from the cell colonies.

15. A method according to one of claims 7 or 8 wherein said protoplasts or cells contain exogenous DNA integrated stably into their genome, which DNA cannot be integrated into the genome by natural means.

16. A method according to claim 15 wherein the integrated exogenous DNA is expressible in plant cells.

17. A method of regenerating graminaceous plants of the subfamily Pooideae, which comprises:
(a) culturing cell cultures according to claim 7 until embryos develop;
(b) culturing the embryos on a medium suitable to induce them to mature and germinate; and
(c) further culturing the resultant plantlets until sufficiently developed to be transferred to soil.

18. A method of regenerating fertile graminaceous plants of the subfamily Pooideae, which comprises:
(a) culturing cell cultures according to claim 7 until embryos develop;
(b) culturing the embryos on a medium suitable to induce them to mature and germinate;
(c) further culturing the resultant plantlets until sufficiently developed to be transferred to soil; and
(d) obtaining seed following controlled or open pollination.

19. A method according to one of claims 17 or 18 wherein said plant cells forming the callus contain exogenous DNA integrated stably into their genome, which DNA cannot be integrated into the genome by natural means.

20. A method according to claim 19 wherein the integrated exogenous DNA is expressible in plant cells.

21. A method for producing transgenic graminaceous plants of the subfamily Pooideae, which comprises:
(a) transforming protoplasts according to claim 1 with exogenous DNA using known transformation methods, which DNA cannot be integrated into the genome by natural means
(b) culturing the transformed protoplasts according to one of claims 7 or 8, and
(c) regenerating complete plants according to one of claims 17 or 18.

22. A method according to claim 21, wherein said exogenous DNA comprises one or more chimeric genes which provide the transformed protoplasts and the protoplast-derived tissues and, in particular, the plants with advantageous properties.

23. A method according to claim 21, wherein said exogenous DNA comprises a non-coding DNA with a regulatory function.

24. A method according to claim 21, wherein a direct gene transfer method is used in step (a) for the transformation of protoplasts.

## Revendications (Revendications pour l'(les) Etat(s) contractant(s) suivant(s) : AT, BE, CH, DE, FR, GB, GR, IT, LI, LU, NL, SE)

1. Culture cellulaire embryogénique, qui dérive de plants de graminées de la sous-famille des Pooideae et peut être isolée à partir des protoplastes, qui régénèrent des parois cellulaires, se divisent et enfin forment un cal, qui peut être régénéré en des plantes entières, et que l'on peut obtenir
(a) en isolant du tissu de parties appropriées de plantes de la sous-famille des Pooideae,
(b) en cultivant ce tissu dans un milieu apte à induire le développement d'un cal embryogénique ou d'embryons;
(c) en déposant périodiquement des cultures des cals susdits et des embryons susdits sur un milieu frais, tandis qu'on transfère de petites grappes cellulaires avec des cellules riches en cytoplasmes dans du milieu frais qui est apte à maintenir en activité une prolifération continue; et
(d) après 0 à 500 transferts, en isolant des grappes cellulaires embryogéniques ayant une taille de 150 /-Lm à 2000 wm.

2. Culture cellulaire embryogénique selon la revendication 1, caractérisée en ce qu'il s'agit, en ce qui concerne les plantes régénérées susdites, de plantes fertiles.

3. Culture cellulaire embryogénique selon la revendication 1, caractérisée en ce qu'il s'agit, en ce qui concerne les plantes de Pooideae susdites, d'herbes ou de céréales à petits grains.

4. Culture cellulaire embryogénique selon la revendication 3, caractérisée en ce qu'il s'agit, en ce qui concerne les herbes susdites, d'herbes choisies parmi les espèces consistant en Poa, Festuca, Lolium, Bromus, Trisetum, Agrostis, Phleum, Alopecurus et Dactylis.

5. Culture cellulaire embryogénique selon la revendication 3, caractérisée en ce qu'il s'agit, en ce qui concerne les céréales à petits grains susdites, de céréales choisies parmi les espèces Avena, Triticum, Secale et Hordeum.

6. Procédé pour l'obtention de protoplastes, qui peuvent être régénérés en des plantes entières, à partir de plants de graminées de la sous-famille des Pooideae, caractérisé en ce qu'on
(a) isole du tissu de parties appropriées de plantes de la sous-famille des Pooideae;
(b) cultive ce tissu dans un milieu qui est en mesure d'induire la formation d'un cal embryogénique ou d'embryons;
(c) dépose des cultures dérivées périodiquement dudit cal et desdits embryons sur un milieu frais, avec transfert de petites grappes de cellules ayant des cellules riches en cytoplasmes dans du milieu frais, qui est apte à maintenir une prolifération continue; et
(d) après 0 à 500 transferts isole des grappes de cellules embryogéniques ayant une taille de 150 µm à 2000 µm; et
(e) au moyen d'une enzyme appropriée, élimine les parois cellulaires et isole les protoplastes résultants.

7. Procédé selon la revendication 6, caractérisé en ce que lesdits protoplastes de Pooideae peuvent être régénérés en des plantes fertiles entières.

8. Procédé selon la revendication 6, caractérisé en ce que le tissu de l'étape (a) est isolé à partir des sections basales de jeunes feuilles se situant à l'intérieur, à partir d'embryons sexuels immatures, à partir d'inflorescences immatures, de semences mûres ou de tissu d'embryons de plantes de la sous-famille des Pooideae.

9. Procédé selon la revendication 8, caractérisé en ce que le tissu de l'étape (a) est isolé à partir des feuilles les plus jeunes, se situant le plus à l'intérieur.

10. Procédé selon la revendication 8, caractérisé en ce que l'isolement des grappes de cellules embryogéniques s'effectue après 0 à 100 transferts.

11. Procédé selon la revendication 10, caractérisé en ce que l'isolement des grappes de cellules embryogéniques s'effectue après 3 à 50 transferts.

12. Procédé pour l'obtention d'une culture de cellules, qui peut être régénérée en des plantes entières, à partir de plants de graminées de la sous-famille des Pooideae, caractérisé en ce que l'on
(a) isole du tissu de parties appropriées de plantes de la sous-famille des Pooideae;
(b) cultive ce tissu dans un milieu qui est en mesure d'induire la formation d'un cal embryogénique ou d'embryons;
(c) dépose des cultures dérivées périodiquement dudit cal et desdits embryons sur un milieu frais, avec transfert de petites grappes de cellules ayant des cellules riches en cytoplasmes dans du milieu frais, qui est apte à maintenir une prolifération continue; et
(d) après 0 à 500 transferts isole des grappes de cellules embryogéniques ayant une taille de 150 /-Lm à 2000 µm; et
(e) éventuellement, au moyen d'une enzyme appropriée, élimine les parois cellulaires et isole les protoplastes résultants;
(f) cultive les protoplastes selon l'étape opératoire (e) ou les cellules végétales selon l'étape opératoire (d) jusqu'à formation de colonies cellulaires dans un milieu de culture approprie;
(g) cultive lesdites colonies cellulaires ou des parties de celles-ci sur un milieu approprié qui favorise la formation de cultures cellulaires; et
(h) isole les cultures cellulaires résultantes.

13. Procédé pour l'obtention d'une culture de cellules selon la revendication 12, qui peuvent être régénérées en plantes entières, à partir de plants de graminées de la sous-famille des Pooideae, caractérisé en ce qu'on
(a) cultive les protoplastes ou les cellules végétales dans un milieu de culture approprié pendant un intervalle de temps suffisant pour favoriser la formation de cultures cellulaires, jusqu'à la formation de colonies de cellules; et
(b) isole les cultures de cellules résultantes.

14. Procédé pour l'obtention d'une culture de cellules selon l'une des revendications 12 ou 13, caractérisé en ce que ladite culture de cellules peut être régénérée en des plantes fertiles entières.

15. Procédé selon l'une quelconque des revendications 12 à 14, caractérisé en ce qu'il s'agit en ce qui concerne ladite culture de cellules d'une culture en suspension.

16. Procédé selon l'une quelconque des revendications 12 à 14, caractérisé en ce qu'il s'agit en ce qui concerne ladite culture de cellules d'une culture de cals.

17. Procédé selon l'une des revendications 12 ou 13, caractérisé en ce qu'on exécute l'étape opératoire (a) en présence d'agarose en tant qu'agent de solidification.

18. Procédé selon la revendication 17, caractérisé en ce qu'on fluidifie ou segmente le milieu de l'étape opératoire (a) renforcé avec de l'agarose et on transfert le milieu d'agarose fluidifié ou segmenté dans un milieu fluide et on y poursuit la culture jusqu'à la formation de colonies cellulaires.

19. Procédé selon l'une des revendications 12 ou 13, caractérisé en ce qu'il s'agit en ce qui concerne lesdites parties d'une colonie de cellules de l'étape opératoire (b) de cellules qui peuvent être libérées à partir des colonies cellulaires.

20. Procédé selon l'une des revendications 12 ou 13, caractérisé en ce que lesdits protoplastes ou cellules contiennent, incorporées de manière stable dans leur génome, de l'ADN exogène qui ne peut pas être intégré de façon naturelle dans le génome.

21. Procédé selon la revendication 20, caractérisé en ce que l'ADN exogène intégré peut s'exprimer dans des cellules végétales.

22. Procédé pour la régénération de plants de graminées de la sous-famille des Pooideae, caractérisé en ce qu'on
(a) cultive des cultures de cellules selon la revendication 12 jusqu'à la formation d'embryons;
(b) cultive les embryons sur un milieu qui est approprié pour induire la maturation et la germination desdits embryons; et
(c) poursuit la culture des jeunes plantules résultantes, jusqu'à ce qu'elles aient atteint un stade de développement permettant un repiquage en terre.

23. Procédé pour la régénération de plants de graminées fertiles de la sous-famille des Pooideae, caractérisé en ce qu'on
(a) cultive des cultures de cellules selon la revendication 12 jusqu'à la formation d'embryons;
(b) cultive les embryons sur un milieu qui est apte à induire la maturation et la germination desdits embryons;
(c) poursuit la culture des jeunes plantules résultantes, jusqu'à ce que soit atteint un stade de développement qui permette un repiquage en terre; et
(d) obtient des semences à la suite d'une pollinisation contrôlée ou libre.

24. Procédé selon l'une des revendications 22 ou 23, caractérisé en ce que lesdites cellules végétales qui forment le cal contiennent de l'ADN exogène incorporé de manière stable dans leur génome et qui ne peut pas être intégré de façon naturelle dans le génome.

25. Procédé selon la revendication 24, caractérisé en ce que l'ADN exogène intégré peut s'exprimer dans des cellules végétales.

26. Procédé pour l'obtention de plants de graminées transgéniques de la sous-famille des Pooideae, caractérisé en ce qu'on
(a) transforme des protoplastes selon la revendication 6 au moyen de procédés de transformation connus avec de l'ADN exogène qui ne peut pas être intégré de façon naturelle dans le génome,
(b) cultive les protoplastes transformés selon l'une des revendications 12 ou 13 et
(c) régénère les plantes entières selon l'une des revendications 22 ou 23.

27. Procédé selon la revendication 26, caractérisé en ce qu'il s'agit, en ce qui concerne ledit ADN exogène, d'un gène chimère ou de plusieurs gènes chimères, qui confèrent aux protoplastes transformés, ainsi qu'aux tissus qui se développent à partir d'eux, et en particulier aux plantes, des propriétés avantageuses.

28. Procédé selon la revendication 26, caractérisé en ce qu'il s'agit, en ce qui concerne ledit ADN exogène, d'un ADN non-codant ayant une fonction régulatrice.

29. Procédé selon la revendication 26, caractérisé en ce qu'on emploie dans l'étape opératoire (a) un procédé de transfert de gène direct pour la transformation des protoplastes.

30. Plants de graminées de la sous-famille des Pooideae, ainsi que leur matériel de reproduction, caractérisés en ce qu'il s'agit, en ce qui concerne ladite plante, d'une plante fertile qui peut être obtenue par régénération à partir d'une culture de cellules embryogéniques selon l'une quelconque des revendications 1 à 5 et dont le matériel de reproduction contient, intégré de manière stable dans le génome des plantes, de l'ADN exogène qui peut s'exprimer dans les plantes ou les cellules végétales et ne peut pas être intégré de façon naturelle dans le génome.

31. Matériel de reproduction selon la revendication 30, caractérisé en ce qu'il s'agit, en ce qui concerne ledit matériel de reproduction, de matériel végétal qui peut être multiplié par voie sexuée ou asexuée, ainsi que in vitro ou in vivo.

32. Matériel de reproduction selon la revendication 31, caractérisé en ce qu'il s'agit, en ce qui concerne ledit matériel de reproduction, de protoplastes, cellules, cals, tissus, organes, zygotes, embryons ou semences, qui proviennent de plantes de Pooideae transgéniques selon la revendication 30.

33. Descendance de plantes selon la revendication 30, dont le génome contient ledit ADN exogène.

## Revendications (Revendications pour l'(les) Etat(s) contractant(s) suivant(s) : ES)

1. Procédé pour l'obtention de protoplastes, qui peuvent être régénérés en des plantes entières, à partir de plants de graminées de la sous-famille des Pooideae, caractérisé en ce qu'on
(a) isole du tissu de parties appropriées de plantes de la sous-famille des Pooideae;
(b) cultive ce tissu dans un milieu qui est en mesure d'induire la formation d'un cal embryogénique ou d'embryons;
(c) dépose des cultures dérivées périodiquement dudit cal et desdits embryons sur un milieu frais, avec transfert de petites grappes de cellules ayant des cellules riches en cytoplasmes dans du milieu frais, qui est apte à maintenir une prolifération continue; et
(d) après 0 à 500 transferts isole des grappes de cellules embryogéniques ayant une taille de 150 I-Lm à 2000 µm; et
(e) au moyen d'une enzyme appropriée, élimine les parois cellulaires et isole les protoplastes résultants.

2. Procédé selon la revendication 1, caractérisé en ce que lesdits protoplastes de Pooideae peuvent être régénérés en des plantes fertiles entières.

3. Procédé selon la revendication 1, caractérisé en ce que le tissu de l'étape (a) est isolé à partir des sections basales de jeunes feuilles poussant à l'intérieur, à partir d'embryons sexuels immatures, à partir d'inflorescences immatures, de graines mûres ou de tissu d'embryons de plantes de la sous-famille des Pooideae.

4. Procédé selon la revendication 3, caractérisé en ce que le tissu de l'étape (a) est isolé à partir des feuilles les plus jeunes, se situant le plus à l'intérieur.

5. Procédé selon la revendication 3, caractérisé en ce que l'isolement des grappes de cellules embryogéniques s'effectue après 0 à 100 transferts.

6. Procédé selon la revendication 5, caractérisé en ce que l'isolement des grappes de cellules embryogéniques s'effectue après 3 à 50 transferts.

7. Procédé pour l'obtention d'une culture de cellules, qui peut être régénérée en des plantes entières, à partir de plants de graminées de la sous-famille des Pooideae, caractérisé en ce que l'on
(a) isole du tissu de parties appropriées de plantes de la sous-famille des Pooideae;
(b) cultive ce tissu dans un milieu qui est en mesure d'induire la formation d'un cal embryogénique ou d'embryons;
(c) dépose des cultures dérivées périodiquement dudit cal et desdits embryons sur un milieu frais, avec transfert de petites grappes de cellules ayant des cellules riches en cytoplasmes dans du milieu frais, qui est apte à maintenir une prolifération continue; et
(d) après 0 à 500 transferts isole des grappes de cellules embryogéniques ayant une taille de 150 /-Lm à 2000 wm; et
(e) éventuellement, au moyen d'une enzyme appropriée, élimine les parois cellulaires et isole les protoplastes résultants;
(f) cultive les protoplastes selon l'étape opératoire (e) ou les cellules végétales selon l'étape opératoire (d) jusqu'à formation de colonies cellulaires dans un milieu de culture approprie;
(g) cultive lesdites colonies cellulaires ou des parties de celles-ci sur un milieu approprié qui favorise la formation de cultures cellulaires; et
(h) isole les cultures cellulaires résultantes.

8. Procédé pour l'obtention d'une culture de cellules selon la revendication 7, qui peuvent être régénérées en plantes entières, à partir de plants de graminées de la sous-famille des Pooideae, caractérisé en ce qu'on
(a) cultive les protoplastes ou les cellules végétales dans un milieu de culture approprié pendant un intervalle de temps suffisant pour favoriser la formation de cultures cellulaires, jusqu'à la formation de colonies de cellules; et
(b) isole les cultures de cellules résultantes.

9. Procédé pour l'obtention d'une culture de cellules selon l'une des revendications 7 ou 8, caractérisé en ce que ladite culture de cellules peut être régénérée en des plantes fertiles entières.

10. Procédé selon l'une quelconque des revendications 7 à 9, caractérisé en ce qu'il s'agit en ce qui concerne ladite culture de cellules d'une culture en suspension.

11. Procédé selon l'une quelconque des revendications 7 à 9, caractérisé en ce qu'il s'agit en ce qui concerne ladite culture de cellules d'une culture de cals.

12. Procédé selon l'une des revendications 7 à 8, caractérisé en ce qu'on exécute l'étape opératoire (a) en présence d'agarose en tant qu'agent de solidification.

13. Procédé selon la revendication 12, caractérisé en ce qu'on fluidifie ou segmente le milieu de l'étape opératoire (a) renforcé avec de l'agarose et on transfert le milieu d'agarose fluidifié ou segmenté dans un milieu fluide et on y poursuit la culture jusqu'à la formation de colonies cellulaires.

14. Procédé selon l'une des revendications 7 ou 8, caractérisé en ce qu'il s'agit en ce qui concerne lesdites parties d'une colonie de cellules de l'étape opératoire (b) de cellules qui peuvent être libérées à partir des colonies cellulaires.

15. Procédé selon l'une des revendications 7 ou 8, caractérisé en ce que lesdits protoplastes ou cellules contiennent, incorporées de manière stable dans leur génome, de l'ADN exogène qui ne peut pas être intégré de façon naturelle dans le génome.

16. Procédé selon la revendication 15, caractérisé en ce que l'ADN exogène intégré peut s'exprimer dans des cellules végétales.

17. Procédé pour la régénération de plants de graminées de la sous-famille des Pooideae, caractérisé en ce qu'on
(a) cultive des cultures de cellules selon la revendication 7 jusqu'à la formation d'embryons;
(b) cultive les embryons sur un milieu qui est approprié pour induire la maturation et la germination desdits embryons; et
(c) poursuit la culture des jeunes plantules résultantes, jusqu'à ce qu'elles aient atteint un stade de développement permettant un repiquage en terre.

18. Procédé pour la régénération de plants de graminées fertiles de la sous-famille des Pooideae, caractérisé en ce qu'on
(a) cultive des cultures de cellules selon la revendication 7 jusqu'à la formation d'embryons;
(b) cultive les embryons sur un milieu qui est apte à induire la maturation et la germination desdits embryons;
(c) poursuit la culture des jeunes plantules résultantes, jusqu'à ce que soit atteint un stade de développement qui permette un repiquage en terre; et
(d) obtient des semences à la suite d'une pollinisation contrôlée ou libre.

19. Procédé selon l'une des revendications 17 ou 18, caractérisé en ce que lesdites cellules végétales qui forment le cal contiennent de l'ADN exogène incorporé de manière stable dans leur génome et qui ne peut pas être intégré de façon naturelle dans le génome.

20. Procédé selon la revendication 19, caractérisé en ce que l'ADN exogène intégré peut s'exprimer dans des cellules végétales.

21. Procédé pour l'obtention de plants de graminées transgéniques de la sous-famille des Pooideae, caractérisé en ce qu'on
(a) transforme des protoplastes selon la revendication 6 au moyen de procédés de transformation connus avec de l'ADN exogène qui ne peut pas être intégré de façon naturelle dans le génome,
(b) cultive les protoplastes transformés selon l'une des revendications 7 ou 8 et
(c) régénère les plantes entières selon l'une des revendications 17 ou 18.

22. Procédé selon la revendication 21, caractérisé en ce qu'il s'agit, en ce qui concerne ledit ADN exogène, d'un gène chimère ou de plusieurs gènes chimères, qui confèrent aux protoplastes transformés, ainsi qu'aux tissus sui se développent à partir d'eux, et en particulier aux plantes, des propriétés avantageuses.

23. Procédé selon la revendication 21, caractérisé en ce qu'il s'agit, en ce qui concerne ledit ADN exogène, d'un ADN non-codant ayant une fonction régulatrice.

24. Procédé selon la revendication 21, caractérisé en ce qu'on emploie dans l'étape opératoire (a) un procédé de transfert de gène direct pour la transformation des protoplastes.
